# EUROPEAN PATENT APPLICATION

(11) **EP 4 674 943 A1**
(43) Date of publication of application: **07.01.2026**
(21) Application number: 24186568.2
(22) Date of filing: 04.07.2024
(51) Int. Cl.: C12M 3/00, C12M 1/12, C12M 3/06, A23L 13/00

(54) **A MEAT CULTURE CONSTRUCT FOR A MEAT CULTURING SYSTEM**

(71) Applicant: Cellular Agriculture Ltd, Llanelli Carmarthenshire SA15 5TL (GB)
(72) Inventor: ARGYLE, Iain, Carmarthenshire, SA15 5TL (GB); ROBERTS, Nigel Somerville, Carnarthenshire, SA15 5TL (GB); ELLIS, Marianne, Carmarthenshire, SA15 5TL (GB); MACBEAN, Alasdair, Carmarthenshire, SA15 5TL (GB); ALLAN, Scott, Carmarthenshire, SA15 5TL (GB); PARRY, Philippa, Carmarthenshire, SA15 5TL (GB); MIRFAKHAR, Moein, Carmarthenshire, SA15 5TL (GB)
(74) Representative: HGF

(57) **Abstract**

The present disclosure provides a meat culture construct (400) for a meat culturing system, the meat culturing system having a housing adapted to receive the meat culture construct. The meat culture construct comprises a plurality of fibres (412) and an end support (404) in which ends of the plurality of fibres are embedded such that the plurality of fibres extend from the end support and into the housing when the meat culture construct is received in the housing of the meat culturing system. At a plane through the end support the plurality of fibres are arranged in a fibre distribution defined by an envelope (414) containing the plurality of fibres. The envelope has a first dimension (416) and a second dimension (418), and a ratio of the first dimension to the second dimension is at least 2:1 in order to enhance fluid access to fibres within the fibre distribution. Also disclosed herein is a harvesting system, a meat culturing system, a method of seeding a meat culture construct, a method of harvesting cultured meat, and a meat of culturing meat.

## Description

### TECHNICAL FIELD

The present invention relates to various aspects of culturing meat, in particular apparatus and methods for seeding, culturing and harvesting processes for meat culturing in a meat culturing system.

### BACKGROUND

Production of cultured meat by the in-vitro culturing of cells of animal origin, such as muscle cells, muscle precursor cells, fat cells and fat precursors, is of increasing interest for food production. Lab-scale production of cultured meat in its simplest form of muscle cells or co-cultures of muscle and fat cells has been achieved, however scaling-up the process to make a viable economic product is still challenging.

It is known to use hollow fibre bioreactors for culturing cells by perfusion, where cell culture media is provided through the lumina of the hollow fibres to feed cells growing on external surfaces of the hollow fibres. Typically, such bioreactors are designed to maximise the number of fibres within the bioreactor by making the fibres as small as possible and by providing a high density of fibres within the bioreactor module. This increases the surface area for growing cells and for nutrient exchange, and so increases the production of cells. Accordingly, bioreactors are typically constructed with small diameter fibres packed at high density.

Due to the large-scale requirements for commercial meat production, it is necessary to improve aspects of cell culturing, as well as the designs of any bioreactor systems used for such culturing.

In particular, there is a need to provide an improved bioreactor that is designed for efficient seeding of muscle cells onto a construct, and which also provides for improved meat culturing, harvesting and collection. There is also a need for the bioreactor to be robust for reliable and economic production of cultured meat at scale.

### SUMMARY OF THE INVENTION

According to an aspect of the present invention, there is provided a meat culture construct for a meat culturing system, the meat culturing system having a housing adapted to receive the meat culture construct,
wherein the meat culture construct comprises a plurality of fibres and an end support in which ends of the plurality of fibres are embedded such that the plurality of fibres extend from the end support and into the housing when the meat culture construct is received in the housing of the meat culturing system, and
wherein at a plane through the end support the plurality of fibres are arranged in a fibre distribution defined by an envelope containing the plurality of fibres, wherein the envelope has a first dimension and a second dimension, and wherein a ratio of the first dimension to the second dimension is at least 2:1 in order to enhance fluid access to fibres within the fibre distribution.

Accordingly, within the meat culture construct the fibres are arranged in an envelope. The envelope may extend along the length of the meat culture construct (in a longitudinal direction perpendicular to the plane of the end support), and the fibres may be maintained within the envelope for the length of the meat culture construct.

In examples, the ratio of the first dimension to the second dimension is at least 2.5:1, for example at least 3:1, for example at least 3.5:1, for example at least 4:1, for example at least 5:1, for example at least 10:1, for example at least 20:1.

Such an aspect ratio between the first dimension and the second dimension creates an envelope of fibres that is relatively thin in one direction (second dimension), allowing greater access and penetration into the middle of the fibre bundle, while also being relatively long in the other direction (first dimension) to provide scale. As explained further hereinafter, seeding, culturing and harvesting are each advantageously improved by aspect ratio of the envelope of the meat culture construct.

In examples, the ratio of the first dimension to the second dimension is at least 50:1, for example at least 100:1, for example at least 200:1, for example at least 300:1, for example at least 500:1, for example at least 500:1.

Advantageously, such ratios provide the advantages mentioned above and additionally provide a larger scale meat culture construct for use in scaled-up manufacturing processes.

In examples, the ratio may be at most 500:1, for example at most 750:1, for example at most 1000:1. In preferred examples the ratio may be between 2:1 and 1000: 1, for example between 2:1 and 500:1, for example between 2:1 and 100:1, for example between 2:1 and 50:1. In examples, the ratio may be between 3:1 and 500:1, for example between 3:1 and 100:1, for example between 3:1 and 50:1. Advantageously, such ratios provide a balance between scale and practicality while still providing a ratio that provides one smaller dimension to improve access and penetration into the fibre bundle.

In examples, the envelope is rectangular. The end support may also be rectangular. The fibres may be evenly or substantially evenly distributed through the envelope.

In examples, the envelope is an annulus formed between an outer boundary and an inner boundary, the second dimension being a distance between the inner boundary and the outer boundary, and wherein the first dimension is a length of the inner boundary or the outer boundary. If, during use, fluid flows from within the annulus to outside the annulus (through the envelope), then the first dimension is the length of the inner boundary. Alternatively if, during use, fluid flows from outside of the annulus to inside the annulus (through the envelope), then the first dimension is the length of the outer boundary. In some examples, as described hereinafter, fluid may flow in both directions during use (e.g., in an alternating direction), in which case both the inner boundary length and outer boundary length may be the first dimension. In examples, the lengths of the inner boundary and outer boundary both satisfy the aspect ratio(s) detailed above, ensuring fluid penetration into the fibres regardless of the direction of fluid flow relative to the annulus.

In examples, the inner boundary and/or the outer boundary is/are circular. The end support may also be circular. In examples, the inner boundary and/or the outer boundary is/are polygonal, for example triangular, square, rectangular, pentagonal, or hexagonal. The end support may have a polygonal shape corresponding to the outer boundary. The fibres may be evenly or substantially evenly distributed through the envelope.

In examples, the end support comprises a seal arranged to seal against a further end support of another meat culture construct when the end support and further end support are arranged adjacent to one another. In this way, multiple meat culture constructs can be arranged side-by-side within the housing of the meat culturing system and seal against each other.

In examples, the second dimension is less than 3cm, for example less than 2.5cm, for example less than 2cm, for example between 1cm and 3cm, for example between 1cm and 2.5cm, for example between 1cm and 2cm, for example between 1.1 cm and 1. 9cm, for example between 1.2cm and 1.8cm, for example between 1.3cm and 1.7cm, for example between 1.3cm and 1.5cm, for example about 1.4cm. Such a small second dimension ensures access and penetration into the fibre bundle, for example by a fluid flow (for seeding, culturing and harvesting) or harvesting apparatus. Additionally, the small second dimension improves cell recovery during harvesting as cells can more easily escape the fibre bundle after being detached, for collection.

In examples, the plurality of fibres are spaced from each other by at least 0.2mm, preferably between about 0.2mm and about 1mm, more preferably between about 0.3mm and about 0.6mm. Such spacings advantageously improve access and penetration into the fibre bundle, for example by a fluid flow or scraper, and also improve recovery of cells during harvesting as detached cells can more easily escape the fibre bundle.

In examples, each fibre of the plurality of fibres has an outer diameter of between 0.2mm and 1.8mm, preferably between 0.3mm and 1.2mm, preferably between 0.5mm and 1.0mm. Such fibres may be suitably robust to be subject to fluid flow and/or deformation and other seeding, culturing and harvesting techniques as set out herein, while also providing a large surface area on which to culture meat.

In examples, the end support comprises a single end support in which the plurality of fibres are embedded. The single end support and fibres can be formed in a single operation, for example potting or sealing the fibres to form the end support.

In other examples, the end support comprises a plurality of end support segments. Each of the end support segments may have a plurality of fibres embedded therein. The plurality of end support segments are arranged to form the end support. In other words, the meat culture construct comprises a plurality of end support segments, each having a plurality of fibres. Advantageously, multiple smaller end support segments can be manufactured (e.g., by potting) and then assembled together to form a larger meat culture construct and end support, allowing greater scales to be achieved with existing manufacturing processes (in particular a centrifuge for forming the end support segments).

In examples, the plurality of end support segments are attached to each other to form the end support. For example, the plurality of end support segments may be potted or adhered together. In another example, the meat culture construct comprises an end plate having a plurality of holes, and the plurality of end support segments are received in the holes. The end support segments may be push-fit or interference-fit in the holes, or may be threaded into the holes. The end plate may be rigid, in particular metal or polymer, or flexible, for example silicone.

In examples, the end support is a first end support and first ends of the plurality of fibres are embedded in the first end support. The meat culture construct may further comprise a second end support, and second ends of the plurality of fibres are embedded in the second end support. In this way, the meat culture construct comprises end supports at each end, and the fibres are supported at each end. Advantageously, this may help to maintain a fibre distribution along the length of the meat culture construct, and allows both ends of the meat culture construct to be held during use.

In examples, the plurality of fibres are hollow fibres, each hollow fibre comprising a lumen. In such examples the first end support (and the second end support if present) comprises a fluid port in fluid communication with lumina of the plurality of hollow fibres. Cells may be adhered and cultured on the outer or inner surfaces of the hollow fibres.

In other examples, the plurality of fibres may be solid fibres. Cells may be adhered and cultured on the outer surfaces of the solid fibres.

In examples, the end support comprises a plurality of fluid outlets for providing a flow of fluid along the meat culture construct, parallel to the plurality of fibres. The plurality of fluid outlets may be connected, for example via a manifold, to a fluid port in the end support. In examples, the fluid outlets may be provided with a gas to form bubbles moving along the lengths of the fibres during use.

According to a further aspect of the present invention there is provided a meat culturing system comprising a housing and one or more of the meat culture constructs described above received in the housing.

In use, the housing holds a fluid, for example a culture medium, and one or more of the meat culture constructs such that the meat culture constructs, in particular the fibres, are immersed in the fluid.

A plurality of meat culture constructs may be disposed in the housing. The meat culture constructs may be tessellated to efficiently fit within the housing. The end supports of the meat culture constructs may abut and optionally seal against each other, or may be held in a frame or end plate of the housing.

In examples, the meat culturing system comprises a seeding station and/or a cell culturing station and/or a harvesting station. Each of the seeding station, cell culturing station and harvesting system may comprise a housing configured to receive the one or more meat culture constructs. The meat culture constructs may be moved between the different stations at different stages of meat culturing. The meat culture constructs may be moved manually, or an automated or robotic transfer system may be provided, for example a conveyor. Within each station the meat culture constructs may have a different spatial arrangement to each other. For example, in the seeding station and the harvesting station the meat culture constructs may be spaced apart to provide fluid access to the sides of the fibres, for seeding and harvesting, respectively. In contrast, in the cell culturing station the meat culture constructs may be more tightly packed against each other to increase production density.

In examples, the meat culturing system further comprises a system housing within which the harvesting system, seeding station and/or cell culturing station are disposed.

In alternative examples, the meat culturing system may be adapted to perform multiple stages of meat culturing (specifically seeding, culturing and/or harvesting) in the same housing. In such examples, the meat culture constructs may be moveable within the housing, for example to space apart or move together the meat culture constructs for performing different processes.

According to another aspect of the present invention there is provided a seeding method for seeding muscle cells and/or muscle cell precursors on the one or more meat culture constructs in the meat culturing system described above, the seeding method comprising introducing a seed cell suspension into the meat culturing system such that the seed cell suspension contacts the one or more meat culture constructs, in particular the fibres.

In examples, the seeding method comprises flowing the seed cell suspension in a direction parallel to the second dimension of the one or more meat culture constructs. Advantageously, the aspect ratio of the envelope allows cells of the seed cell suspension to more easily penetrate the fibre bundle in the flow direction, creating a more even distribution of cells across the fibres.

In examples, the fibres are hollow fibres having lumina, and the seeding method comprises introducing the seed cell suspension into the housing of the meat culturing system and applying suction to the lumina of the hollow fibres to draw cells of the seed cell suspension against the outer surfaces of the hollow fibres. Advantageously, the suction draws cells against the outer surfaces of the fibres, improving cell adhesion, and the fluid flow generated by the suction will also improve the evenness of the cell distribution across the fibre surfaces.

In examples, prior to the seeding method the muscle cells and/or muscle cell precursors are:
i. washed with a composition comprising a cell detachment agent;
ii. harvested in a culture media comprising a growth promotion agent;
iii. centrifuged to form a pellet; and
iv. resuspended in a culture media to form a cell suspension.

The cells seeded onto the fibres include muscle cells and/or muscle cell precursors. Muscle cells include those cells making up contractile tissue of animals or cells that can differentiate into muscle cells. Muscle cells are derived from the mesodermal layer of embryonic germ cells. Mature muscle cells contain contractile filaments that move past each other and change the size of the cell. They are classified as skeletal, cardiac, or smooth muscles. As used herein, the term "cells that can differentiate into muscle cells" and "muscle cell precursors" refers to stem cells and muscle progenitor cells that can differentiate into muscle cells (e.g. mature muscle cells).

Muscle cells may include those cells normally found in muscle tissue, including smooth muscle cells, cardiac muscle cells, skeletal muscle cells (e.g., muscle fibres or myocytes, myoblasts, myotubes, etc.), and any combination thereof. Muscle cells may include myoblasts, myotubes, myofibrils, and/or satellite cells.

The cells may further include adipose or fat cells. Adipose or fat cells include any cell or group of cells composed in a fat tissue, including, for example, lipocytes, adipocytes, adipocyte precursors including, pre-adipocytes and mesenchymal stem cells.

The cells may be derived from any source animal. As the bioreactor systems described herein may be for use in making comestible products, the cells may not be derived from a human. In some examples, the cells may be derived from bovine, ovine, equine, porcine, caprine, avian, fish, insect, crustaceans, cephalopod, mollusc and/or camelid animals. Preferably the cells may be derived from a bovine, porcine, avian and/or ovine animal. For example, the cells may be derived from a cow, pig, chicken, fish, squid, insect, oyster and/or sheep.

As used herein, the term "cells" refers to any one or more of the above types of cells.

According to another aspect of the present invention there is provided a method of culturing meat on the one or more meat culture constructs in the meat culturing system described above. The method of culturing meat comprises comprising introducing a cell culture medium into the meat culturing system such that the cell culture medium contacts the one or more meat culture constructs to supply nutrients to the muscle cells and/or muscle cell precursors seeded thereon.

In examples, the method of culturing meat comprises flowing the cell culture medium in a direction parallel to the second dimension of the one or more meat culture constructs. Advantageously, the nutrients of the cell culture medium can more easily penetrate the fibre bundle in the flow direction, providing improved nutrient supply to the cells being cultured.

In examples, the fibres are hollow fibres having lumina, and the method of culturing meat comprises introducing the cell culture medium into the housing of the meat culturing system and flowing the cell culture medium suction against the outer surfaces of the hollow fibres.

In another example, the meat culture construct may comprise an annular envelope, as described above, the method of culturing meat may comprise flowing the cell culture medium through the central channel of the meat culture construct and into the housing via the envelope, or in the opposite direction.

According to another aspect of the present invention there is provided a harvesting system comprising a housing and one or more of the meat culture constructs described above received in the housing.

In use, the housing holds a fluid, for example a culture medium, and one or more of the meat culture constructs such that the meat culture constructs, in particular the fibres, are immersed in the fluid.

In examples, the one or more meat culture constructs are removable from the housing. A plurality of meat culture constructs may be individually removable from the housing. Advantageously, this may allow for different meat culture constructs to be added to, and removed from, the system at different times.

In examples, the harvesting system further comprises a fluid supply system arranged to generate a fluid flow through the housing in a direction parallel to the second dimension of the one or more meat culture constructs. The fluid flow may act to detach cells from the fibres, for example by shearing the cells from the fibre surfaces, and/or by deforming the fibres to encourage detachment. Advantageously, the fluid flow can more easily penetrate the fibre bundle in the flow direction (due to the aspect ratio of the envelope), improving detachment of cells. In addition, the fluid flow may entrain detached cells, allowing the detached cells to be conveyed to a collection point in the fluid flow.

In examples, the plurality of fibres are hollow fibres, each hollow fibre comprising a lumen. In such an example the harvesting system may further comprise a fluid supply system arranged to provide a fluid flow into and/or through the lumina of the plurality of hollow fibres. If cells are adhered to the outer surfaces of the fibres the fluid flow would flow into the lumen, permeate through the walls of the fibres, and help to detach cells adhered to the outer surfaces of the fibres. In such an example the detached cells would be entrained in a fluid flow in the housing, which may be extracted for cell collection. In other examples, if the cells are adhered to the inner surfaces of the fibres, then the fluid flow would push the cells along the lumina, where they can be collected at the end of the meat culture construct.

In examples, the harvesting system further comprises a detachment fluid supply system arranged to provide a flow of detachment fluid into and/or through the housing and/or the meat culture construct, in particular through lumina of hollow fibres. The detachment fluid may comprise a detachment agent, for example a chemical or biochemical detachment agent. In examples, the flow of detachment fluid may be in a direction parallel to the second dimension of the one or more meat culture constructs, as described above, thereby improving penetration of the detachment agent into the fibre bundle. In addition, the flow of detachment agent may entrain detached cells, allowing the detached cells to be conveyed to a collection point.

In examples, the harvesting system may further comprise a cell collection system. The cell collection system may be configured to filter and/or concentrate the cells. The cell collection system may be arranged to receive a fluid flow from the housing.

In examples, the harvesting system further comprises a heating and/or cooling system for heating and/or cooling the one or more meat culture constructs within the housing. Advantageously, heating and/or cooling may apply thermal shock to the cells, assisting with detachment by causing the cells to contract or otherwise change shape. In examples, heating the cells may cause stiffening of the cells (particularly stiffening of collagen in the cells), making it easier to remove the cells by a physical detachment technique such as a shearing fluid flow or scraping.

In examples, the harvesting system further comprises an electromagnetic emitter (EM emitter). The EM emitter may be configured to irradiate the meat culture constructs with electromagnetic radiation (EM radiation), for example visible light, UV light, or other EM radiation. The EM radiation may cause cells to detach from the meat culture constructs or weaken their adhesion to the fibres.

In examples, the harvesting system further comprises an actuator arranged to move the end support of one or more of the meat culture constructs relative to the another end of the or each meat culture construct. Such movement may be linear or rotary, and may be reciprocal or oscillatory. Such movement may apply vibrations and/or shaking to the fibres to encourage or cause cell detachment. In examples, the movement acts to spread apart the plurality of fibres, improving fluid access and penetration into the fibre bundle and allowing more cells to escape the fibre bundle for collection. In examples, the or each meat culture construct comprises a first end support and a second end support holding opposite ends of the fibres, and the harvesting system may comprise end plates or the like that hold each of the first end support and the second end support. At least one of the end plates may be moveable to provide the motion.

In examples, the harvesting system further comprises a rotating ball device arranged to generate one or more fluid jets and move relative to the one or more meat culture constructs for detaching cells therefrom. In examples where the envelope of the meat culture construct is annular, the meat culture construct may include a central channel. The rotating ball device may be moved within the central channel such that the fluid jets act in a direction parallel to the second dimension of the envelope.

In examples, the harvesting system further comprises a scraper arranged to move relative to the or each meat culture construct for scraping cells therefrom. The scraper may be moved in longitudinal direction of the fibres or across the fibres. Advantageously, the scraper may contact a side of the fibre bundle that is parallel to the first dimension, such that the scraper has improved access and penetration into the fibre bundle. In examples, the scraper comprises a flat scraper edge that is moved along the fibres. In examples, the scraper comprises a fluid knife arranged to generate a fluid flow and move relative to the one or more meat culture constructs for detaching cells therefrom. In other examples, the scraper comprises a harvesting comb having a plurality of teeth arranged to contact the one or more meat culture constructs and move relative to the or each meat culture construct for detaching cells therefrom.

In examples, the harvesting system further comprises a centrifuge for rotating the or each meat culture construct for detachment of cells therefrom. The centrifuge may additionally or alternatively be used to assist in cell collection after cells have been detached.

According to a further aspect of the present invention there is provided a harvesting method for harvesting muscle cells from the meat culture construct of the harvesting system described above, the harvesting method comprising detaching the muscle cells from the plurality of fibres, and collecting the detached muscle cells.

In examples, detaching the muscle cells comprises providing a chemical or biochemical detachment agent. The chemical or biochemical detachment agent may be provided in the housing, and may be provided as a fluid flow flowing in a direction parallel to the second dimension of the one or more meat culture constructs. In other examples, particularly where the meat culture construct comprises hollow fibres, the chemical or biochemical detachment agent may be flowed into and/or through the lumina of the meat culture construct.

In examples, the chemical or biochemical detachment agent comprises at least one of:
chelators, for example EDTA, EGTA, citric saline, citric acid salts e.g. sodium citrate, as well as proprietary singular or blended products, for example, Chemical Dissociation Buffer; and
an enzymatic detachment agent, for example a protease, for example trypsin.

In examples, detaching and/or collecting the muscle cells comprises applying a physical detachment technique.

In examples, the physical detachment technique comprises providing a fluid flow in a direction parallel to the second dimension of the one or more meat culture constructs. The fluid flow may act to detach the cells from the fibres, for example by shearing. By providing the fluid flow parallel to the second dimension, penetration of the fluid flow into the fibres is improved, thereby improving harvesting.

In examples, the plurality of fibres comprise hollow fibres and cells are adhered to external surfaces of the hollow fibres. In such an example the fluid flow may be provided through the housing in a direction parallel to the second dimensions of the one or more meat culture constructs.

In other examples, the fluid flow is provided into and/or through the lumina of the hollow fibres. In examples, the fluid flow through the lumina of the hollow fibres may deform the hollow fibres. Specifically, providing the fluid flow through the lumina of the hollow fibres may comprise providing a fluid flow with a pressure sufficient to deform the hollow fibres. Deforming the fluid fibres may cause cells to detach.

In examples, the meat culture construct comprises an annular envelope with a central channel, and the fluid flow is provided from the housing into the central channel, and/or vice versa, through the envelope. In this way, the fluid flow is parallel to the second dimension and would act to detach cells from the fibres.

In some examples the direction of the fluid flow is reversed, for example periodically or regularly reversed. In some examples, the fluid flow has a variable pressure. In these examples, changing the fluid flow (direction and/or pressure) may induce additional shearing forces to detach cells.

In examples, the harvesting method comprises deforming the or each meat culture construct to spread apart the fibres, and providing a chemical or biochemical detachment agent and/or a fluid flow. Advantageously, spreading apart the fibres further improves access and penetration of the detachment agent and/or fluid flow into the fibre bundle, improving cell detachment and harvesting.

In examples, the physical detachment technique comprises emitting electromagnetic radiation onto the one or more meat culture constructs. The electromagnetic radiation may comprise visible light, UV light, or other EM radiation. The EM radiation may cause cells to detach from the meat culture constructs.

In examples, the physical detachment technique comprises heating and/or cooling the one or more meat culture constructs. Advantageously, heating and/or cooling may apply thermal shock to the cells, assisting with cell detachment. In examples, heating the cells may cause stiffening of the cells (particularly stiffening of collagen in the cells), making it easier to remove the cells by a physical detachment technique such as a shearing fluid flow or scraping.

In examples, the physical detachment technique comprises generating an electric field in the housing and/or through the fibres. The electric field may reduce cell-fibre adhesion and/or cause them to detach from the fibres.

In examples, the physical detachment technique comprises applying motion to the one or more meat culture constructs, for example vibration, shaking or oscillatory motion. Such motion may cause cells to detach from the fibres, and/or free detached cells that are otherwise trapped within the fibre bundle, allowing them to be collected.

In examples, the physical detachment technique comprises moving the end support of the or each meat culture construct relative to an opposite end of the or each meat culture construct to deform the fibres. Such movement may be linear or rotary, and may be reciprocal or oscillatory. Such movement may apply vibrations and/or shaking to the fibres to encourage or cause cell detachment. In examples, the movement acts to spread apart the plurality of fibres, improving fluid access and penetration into the fibre bundle and allowing more cells to escape the fibre bundle for collection.

In examples, the physical detachment technique comprises applying sonication, for example ultrasonication, to the one or more meat culture constructs. Sonication may be applied continuously or intermittently to agitate cells by promoting shockwaves that detach cells. The shockwaves may create cavitation or other phenomena that further improves cell detachment.

In examples, the physical detachment technique comprises centrifuging the or each meat culture construct. Centrifugation may assist cell detachment and collection at one end or port of the meat culturing system.

In examples, the physical detachment technique comprises scraping, for example combing, cells from the one or more meat culture constructs. A scraper may be moved in the longitudinal direction of the fibres or across the fibres. Advantageously, the scraper may contact a side of the fibre bundle that is parallel to the first dimension, such that the scraper has improved access and penetration into the fibre bundle. In examples, the scraper comprises a flat scraper edge that is moved along the fibres. In examples, the scraper comprises a fluid knife arranged to generate a fluid flow and move relative to the one or more meat culture constructs for detaching cells therefrom. In other examples, the scraper comprises a harvesting comb having a plurality of teeth arranged to contact the one or more meat culture constructs and move relative to the or each meat culture construct for detaching cells therefrom.

In examples, the physical detachment technique comprises imparting a fluid jet on the one or more meat culture constructs. In examples, the fluid jet is provided by a rotating ball device. In examples where the envelope of the meat culture construct is annular, the meat culture construct may include a central channel. The rotating ball device may be moved within the central channel such that the fluid jets act in a direction parallel to the second dimension of the envelope.

In examples, the physical detachment technique comprises flowing gaseous bubbles over the fibres of the one or more meat culture constructs. Gaseous bubbles may be created an fluid openings in the end support of each meat culture construct, the fluid outlets being parallel to and interspersed with the fibres. The gaseous bubbles may flow along the lengths of the fibres. The gaseous bubbles may comprise air, nitrogen, carbon dioxide, oxygen or other gas.

In examples, the harvesting method comprises providing a chemical or biochemical detachment agent to the or each meat culture construct and providing a fluid flow to detach cells from the plurality of fibres. Advantageously, the detachment agent may detach some cells and weaken the adherence of other cells, allowing further cells to be detached by the fluid flow. In addition, the fluid flow may entrain the detached cells and convey them to a collection point or collection system.

In examples, the fibres are hollow fibres with lumina, and the cells are adhered to outer surfaces of the hollow fibres. In this example, the harvesting method comprises flowing an enzymatic detachment agent through the lumina of the hollow fibres under pressure such that a fluid flow is generated from the lumina into the housing. In this way, the enzymatic detachment agent permeates through the walls of the fibres and is incident directly on the cell-fibre adhesion site, causing detachment of the cells. In addition, this would create a fluid flow into the housing, which can be extracted at a collection point, for example a fluid outlet port.

In examples, the harvesting method comprises providing a protease enzymatic detachment agent to the or each meat culture construct and subsequently subjecting the or each meat culture construct to one or more of vibration, shaking, deformation or centrifugation. In this way, the protease enzymatic detachment agent may detach some cells and weaken the adherence of other cells, allowing further cells to be detached by the vibration, shaking, deformation of centrifugation.

In examples, the harvesting method comprises providing an enzymatic detachment agent to the or each meat culture construct and subsequently subjecting the or each meat culture construct to sonication. In this way, the enzymatic detachment agent may detach some cells and weaken the adherence of other cells, allowing further cells to be detached by the sonication.

In examples, the harvesting method comprises heating the or each meat culture construct and subsequently scraping and/or combing cells from the or each meat culture construct. Advantageously, heating the meat culture constructs induces thermal shock in the cells, causing at least partial detachment. Additionally, continuing to heat the meat culture constructs may stiffen the cells by stiffening collagen within the cells, akin to cooking. This allows the cells to be more easily detached by scraping and/or combing.

According to a further aspect of the present invention there is provided a method of culturing meat, the method comprising:
seeding a meat culture construct with muscle cells and/or muscle cell precursors by the seeding method described above,
culturing the muscle cells and/or muscle cell precursors to form a cultured meat, and
harvesting the cultured meat.

In examples, seeding the meat culture construct may comprise the seeding method described above. In examples, harvesting the cultured meat may comprise the harvesting method described above.

Culturing the muscle cells and/or muscle cell precursors may comprise flowing a cell culture medium through the housing to supply the muscle cells and/or muscle cell precursors with nutrients. The flow of the cell culture medium may be in a direction parallel to the second dimension of the one or more meat culture constructs, thereby improving supply of nutrients to the muscle cells and/or muscle cell precursors by improving penetration of the cell culture medium into the fibre bundle.

In examples, harvesting the cultured meat is performed after one or more of:
a predetermined culturing time or maturation time has elapsed,
the cultured meat reaches a predetermined size and/or mass during culturing,
a monitored parameter of a cell culture medium during culturing reaches a predetermined threshold, and
a detected cell maturity marker reaches a predetermined threshold during culturing.

Advantageously, harvesting matured cells may be beneficial as the cells will have naturally reduced their adhesion to the fibres due to contractile prestresses within the cells (so-called maturation forces). Accordingly, it may be beneficial to prolong the culturing process to allow more of the cells to reach maturity in order to improve the harvesting process.

A cell is considered "mature" once it stops differentiating. A maturation medium may be provided to mature the cells before harvesting. One or more cell maturation markers may be detected / monitored to determine when a proportion or majority or all of the cells are mature and harvesting can be initiated.

Different markers may be detected as the method of culturing meat progresses to determine the state of differentiation / proliferation / maturation of the cells. Such markers may be detected by antibody labelling, as is known in the art.

Examples of markers for proliferating myoblasts include PAX3, c-Met, Mox2, MSX1, Six1/4, Myf5, and/or MyoD.

Examples of markers for differentiating muscle cells include Myogenin, MCF2, Six1/4, MyoD, Myf6.

Examples of markers for muscle formation (myotube formation) include Lbx1, and Meox2.

Examples of markers for differentiated or mature skeletal muscle cells include: MYH2, FABP3, Integrin alpha 7, MYH2, alpha-Sarcoglycan, beta-Sarcoglycan, Calpain Inhibitors, Creatine Kinase MM/CKMM, eIF5A, Enolase 2, Neuron-specific Enolase, epsilon-Sarcoglycan, GDF-8, Myostatin, GDF-11, GDF-8, Integrin alpha 7 beta 1, Integrin beta 1, MCAM, MyoD, Myogenin, Myosin Light Chain Kinase Inhibitors, NCAM-1, Troponin I, and/or Troponin I/TNNI3; and
Examples of markers for differentiated or mature smooth muscles cells include: VE-Cadherin, alpha-smooth muscle actin, Caldesmon, Calponin 1, Desmin, Histamine H2 R, Motilin R Transgelin, and/or Vimentin.

An example of a marker for mature myotube may be MYH2 (myoglobin heavy chain 2).

In certain embodiments, circulating the cell culture medium comprises pumping culture media through the meat culturing system (either through the internal lumina of the hollow fibres, or through the housing). The cell culture media may be circulated at a rate of at least 10µL/hour/fibre.

In particular, in some examples where the meat culture construct has hollow fibres, the cell culture media is circulated through the lumina of the hollow fibres. Nutrients (e.g., oxygen) in the cell culture media are transferred to cells growing on the outside of the hollow fibres by diffusion. In other examples where the meat culture construct has hollow fibres, the cell culture media is additionally or alternatively circulated through the housing. In other examples where the meat culture construct has solid fibres, the cell culture media is circulated through the housing.

In certain embodiments, the method also includes maintaining the meat culture construct (and cells) at a temperature of at least 15°C and/or 5% CO₂.

In certain embodiments, the cell culture media is circulated through the meat culturing system for at least 3 hours.

In certain embodiments, a first culture media is first circulated through the meat culturing system, optionally wherein the first culture media is a proliferation medium.

In certain embodiments, the method further comprises circulating a second cell culture media through the meat culturing system, optionally wherein the second cell culture media is a differentiation medium; further optionally wherein the differentiation media is circulated through the meat culturing system for at least 3 hours.

In certain embodiments, the meat culturing system comprises apparatus for monitoring metabolite concentration and/or oxygen concentration of the culture media.

In certain embodiments, the harvested cultured meat is formed into a cultured meat product; or the one or more fibres are edible, and the harvested cells and the one or more fibres are formed into a cultured meat product.

The cell culture medium that may be used in the methods described herein may be any suitable cell culture medium. The cell culture medium may be selected depending on the type of cell cells being cultured. Examples of culture mediums that may be used include minimal essential medium (MEM, Sigma, St. Louis, Mo); Dulbecco's modified Eagle medium (DMEM, Sigma); Ham F10 medium (Sigma); Cell culture media (HyClone, Logan, Utah); RPMI-1640 culture media (Sigma); and chemical-defined (CD) culture media (which are formulated for individual cell types), such as CD-CHO culture media (Invitrogen, Carlsbad, Calif). In examples, the cell culture media may be pharmaceutical-grade, or food-grade media. The culture solution described above can be supplemented with auxiliary components or contents as needed, for example to change the properties or behaviours of the cells. This includes any component of the appropriate concentration or amount required or desired.

In some examples, the cell culture media may be configured to control, for example to facilitate, proliferation, differentiation, and/or maturation. Different media and/or physical stimuli may be used at different stages of the cell culturing process.

The culture medium described above can be supplemented with auxiliary components or contents as needed. The culture medium may include one or more additives such as antibiotics, proteins, amino acids and/or sugars.

"Medium" and "cell culture medium" refer to a nutrient source used for growing or maintaining cells. As is understood by a person of skill in the art, the nutrient source may contain components required by the cell for growth and/or survival or may contain components that aid in cell growth and/or survival. Vitamins, essential or non-essential amino acids, trace elements, and surfactants (e.g., poloxamers) are examples of medium components. Any media provided herein may also be supplemented with any one or more of insulin, plant hydrolysates and animal hydrolysates.

"Culturing" a cell refers to contacting a cell with a cell culture medium under conditions suitable for the viability and/or growth and/or proliferation, differentiation and maturation of the cell.

Perfusing the cell culture medium may include perfusing a first culture media and then subsequently perfusing one or more second cell culture mediums. The first cell culture medium may be a cell culture medium that is for proliferating cells and may be referred to as proliferation medium.

Proliferation medium may be a medium comprising a source of nutrients, such as vitamins, minerals, carbon and energy sources, and other beneficial compounds that facilitate the biochemical and physiological processes occurring during expansion or proliferation of cells. The proliferation medium may comprise one or more carbon sources, vitamins, amino acids, and inorganic nutrients. Representative carbon sources include monosaccharides, disaccharides, and/or starches. For example, the proliferation medium may contain one or more carbohydrates such as sucrose, fructose, maltose, galactose, mannose, and lactose. The proliferation medium may also comprise amino acids. Suitable amino acids may include amino acids commonly found incorporated into proteins as well as amino acids not commonly found incorporated into proteins, such as arginosuccinate, citrulline, canavanine, ornithine, and D-stereoisomers. The proliferation medium may also comprise growth promotion agents, such as serum, for example, foetal bovine serum (FBS). Examples of other growth promotion agents include growth factors (e.g. recombinant growth factors), bovine ocular fluid, sericin protein, earthworm heat inactivated coelomic fluid. In some examples, the proliferation media may be a serum free culture media and may optionally include additional components depending on the cells being cultured. For example, serum free culture medias include those commercially available from ThermoFisher, Lonza Bioscience and Merck. The proliferation medium may also comprise antibiotics.

For example, the proliferation medium may be Dulbecco's Modified Eagle's Medium (DMEM), which may include 10% (V/V) filter sterilised foetal bovine serum and 1% (V/V) penicillin/streptomycin solution.

In some examples, such as when the cells are derived from an insect, the proliferation media may be a media such as Gibco insect media available from ThermoFisher.

The cells may be maintained and cultured in proliferation medium for at least 3 hours. For example at least 3, 4, 5, 6, 7, 8, 9, 10, 11, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 125, 130, 135, 140, 145 or 150 hours. In some examples, the cells may be maintained in proliferation media continuously.

The cell culture media may be changed to a second cell culture medium. The second cell culture medium may be a differentiation medium. Differentiation medium refers to a medium designed to support the differentiation of cells, that is, supporting the process of a cell changing from one cell type to another. The differentiation medium may include one or more amino acids, antibiotics, vitamins, salts, minerals, or lipids. The differentiation medium may include at least one carbon source, such as a sugar. For example, glucose. The differentiation medium may include one or more proteins, amino acids or other additional acids. In some examples, the differentiation media includes one or more growth promotion agents, such as serum, for example, foetal bovine serum or horse serum. Examples of other growth promotion agents include growth factors (e.g. recombinant growth factors), bovine ocular fluid, sericin protein, earthworm heat inactivated coelomic fluid. In some examples, the differentiation media may be a serum free culture media and may optionally include additional components depending on the cells being cultured. For example, serum free culture medias include those commercially available from ThermoFisher, Lonza Bioscience and Merck. In some examples, the differentiation medium may be high-glucose DMEM (97%) supplemented with 2% horse serum and 1% penicillin/streptomycin solution.

The cells may be maintained and cultured in differentiation medium for at least 3 hours. For example at least 3, 4, 5, 6, 7, 8, 9, 10, 11, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 125, 130, 135, 140, 145 or 150 hours.

In some examples, the culture media may be changed a number of times. For example, the cells may first be cultured in proliferation media; then, the culture media is switched to differentiation media. The differentiation media may then be switched to proliferation media. The proliferation media may be the same as before or may contain different components and/or concentrations of components to the initial proliferation media.

In some examples, the cells may be continuously cultured. That is to say that the cells are maintained in one or more culture mediums as described herein. The cells may be cultured for a first time period (e.g. at least 3 hours or more) in a first media, then for a second time period (e.g. at least 3 hours or more) in a second media and then cultured in a further media and continuously cultured with any number of changes of the culture media so that cells can be constantly proliferated and/or differentiated. In such examples, the cells may be harvested at predetermined cell densities or time points in order to avoid overcrowding, reduced viability and/or cell death.

In examples, the cell culturing process may be manual, automated, or semi-automated. In particular, the addition and changing of cell culture media may be carried out manually, or in an automated or semi-automated manner.

The cell culturing conditions (e.g. for either or both proliferation or differentiation) may be selected depending on the cell type and/or cell source. In some examples, cells may be cultured (e.g. proliferated and/or differentiated) at a temperature of at least 5°C. In some examples, the cells may be cultured at a temperature of at least 5°C, 10°C, 15°C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, or 37°C.

For example, for culturing of insect cells, the cells may be cultured at a temperature of from 15°C to 32 °C.

For example, for culturing mammal cells, the cells may be cultured at a temperature of 37°C.

For example, for marine animal cells (such as crustaceans, fish or mollusc cells), cells may be cultured at a temperature of from 15°C to 32 °C. In some examples, marine animal cells may be cultured at a temperature between 15°C to 30 °C.

The cells may be cultured in defined atmospheric conditions. For example, the cells may be cultured in an atmosphere that has predetermined humidity and/or gas concentration. For example, cells may be cultured in an atmosphere including at least 5% CO2.

In examples, the method may comprise a wash and/or sterilisation step preceding the seeding of cells to the meat culture construct.

In another aspect of the invention there is provided a comestible product comprising cultured meat (in particular muscle cells) obtained by a method as described above. The comestible product may be a cultured meat product.

A cultured meat product is a meat product comprising fragments of cultured meat along with flavouring and other additives. The cultured meat product may be mixed with other sources of protein, such as plant proteins.

In other examples, the meat culturing system and meat culture construct may be used to culture other types of cells, particularly other types of adherent cells.

As described above, the fibres may be hollow or solid fibres. In examples, the hollow fibres may be porous.

In certain embodiments, the one or more fibres comprise a polymer. In certain embodiments, the one or more fibres are biodegradable and/or edible.

Polymers that may be used to form the fibres may be any polymer suitable for culturing and/or maintenance of cells. Suitable polymers include biodegradable polymers. The polymer may be a biocompatible polymer. Biodegradable polymers are any polymers that may be broken down by biological systems, such as polymers that can be broken down into harmless products by the action of living organisms. Biocompatible polymers are, along with any metabolites or degradation products thereof, generally nontoxic to cells or to a recipient (such as a human or animal) and do not cause any significant adverse effects to cells or a recipient at concentrations resulting from the degradation of the polymers. Generally speaking, biocompatible polymers are polymers that do not elicit result in negative effects on cell health or in a recipient. As one use for the fibres described herein is the production of comestible products, biocompatible and/or biodegradable polymers may be advantageous if the fibres or part thereof is consumed (for example, ingested) by a person.

Biodegradable polymers include linear aliphatic polyesters such as polylactic acid, polyglycolic acid, polycaprolactone, polyhydroxybutyrate, polyhydroxyvalerate and their copolymers within the aliphatic polyester family such as poly(lactic-co-glycolic acid) and poly(glycolic acid-co-caprolactone); copolymers of linear aliphatic polyesters and other polymers such as poly(glycolic acid-co-trimethylene carbonate) copolymers, poly(lactic acid-co-lysine) copolymers, tyrosine-based polyarylates or polyiminocarbonates or polycarbonates, poly(lactide-urethane) and poly(ester-amide) polymers; polyanhydrides such as poly(sebacic anhydride); polyorthoesters such as 3,9-diethyidiene-2,4,8,10-tetraoxaspiro-5,5-undecane based polymers; poly(ester-ether) such as poly-p-dioxanone; polyamides, poly(amide-enamines) and poly(amido amine) dendrimers; and phosphorus-based polymers such as polyphosphazene and poly[bis(carboxy-lactophenoxy)] phosphazene.

The polymers may be edible polymers. Edible polymers refers to any polymer that is acceptable for use in an edible product.

Examples of edible polymers include polyvinyl alcohol, carboxyvinyl polymer, hydroxypropylmethylcellulose, hydroxyethylcellulose, methylcellulose, ethylcellulose, low-substituted hydroxypropylcellulose, crystalline cellulose, carboxymethylcellulose sodium, a synthetic polymer compound such as carboxymethylcellulose calcium, carboxymethylcellulose and carboxymethylstarch sodium, sodium alginate, dextran, casein, pullulan, pectin, guar gum, xanthan gum, tragacanth gum, acacia gum, zein, gelatin, chitin and chitosan, silk, fibrin and polymer compounds obtained from natural products such as starch or soybean. Edible polymers may include edible proteins from animal or plant sources. For example such edible proteins may be derived from legumes such as chickpea or pea. Edible polymers may also be derivable from waste food such as potatoes.

The use of an edible polymer may provide fibres and products, including the fibres which are edible. For example, a cell culture grown on the fibres may provide for an edible product that does not require the removal of the fibres before consumption.

The fibres may be a digestible polymer. "Digestible" refers to a material that, when eaten by a subject, can be broken down into compounds that can be absorbed and used as nutrients or eliminated by the subject's body. Digestible polymers include BCS, polylactic acids (PLAs), synthetic polyamides, polycarbonates, polyisocyanurates (PIRs), polyurethanes, polyethers, proteins, polysaccharides (such as starches), polylactones, polylactams or glycols.

In some examples, the fibres include poly(lactic-co-glycolic acid) (PLGA). For example, the fibres may include 10% PLGA.

In some examples, the fibres include a reusable polymer. A reusable polymer refers to a polymer that does not degrade over time or due to use in culturing cells. Reusable polymers may provide a meat culture construct that can be washed and used multiple times. In some examples, the fibres are hydrophobic. In some examples, the fibres are washable. Washable refers to hollow fibres that can be washed and/or sterilised without sustaining damage or loss of function. Fibres with such properties may reduce waste and costs.

In some examples, the polymer may comprise cellulose or cellulosic polymers or mixtures of both, polysulfone, polypropylene, acrylonitrile butadiene styrene (ABS), polycarbonate, polyethylene, or mixtures thereof. Suitable polymers include biodegradable polymers. Biodegradable polymers are any polymers that may be broken down by biological systems, such as polymers that can be broken down into harmless products by the action of living organisms.

In some examples, the polymer comprises polystyrene. The polymer can comprise polystyrene and a second polymer. The second polymer can be selected from the group consisting of polycarbonate, polypropylene, polyvinylidene fluoride, polycaprolactone, polysulfone, cellulose acetate, cellulose triacetate, polyethersulfone, polyphenylenesulfon, polymethyl methacrylate, acrylonitrile butadiene styrene or mixtures thereof. The composition can comprise polystyrene and a second polymer in a wt. % ratio of about 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 or 95:5.

In some examples, the polymer comprises polysulfone. The polymer can comprise polysulfone and a second polymer. The second polymer can be selected from the group consisting of polystyrene, polycarbonate, polypropylene, polyvinylidene fluoride, polycaprolactone, cellulose acetate, cellulose triacetate, polyethersulfone, polyphenylenesulfon, polymethyl methacrylate, acrylonitrile butadiene styrene or mixtures thereof. The composition can comprise polysulfone and a second polymer in a wt. % ratio of about 5:95 to about 95:5 (e.g. about 10:90 to about 90:10), more preferably about 20:80 to about 80:20, and even more preferably about 25:75 to about 75:25. Polysulfone and the second polymer may be present in a wt. % ratio of 30:70, 35:65, 40:60, 45:55, 50:50, 55:45, 60:40, 65:35, 70:30.

Preferably, the polymer comprises polystyrene and/or polysulfone. Preferably, the polymer comprises polystyrene and polysulfone in a wt. % ratio of about 5:95 to about 95:5 (e.g. about 10:90 to about 90:10), more preferably about 20:80 to about 80:20, and even more preferably about 25:75 to about 75:25. Polystyrene and polysulfone may be present in a wt. % ratio of 30:70, 35:65, 40:60, 45:55, 50:50, 55:45, 60:40, 65:35, 70:30. Preferably, the composition comprises about 80 wt. % of polystyrene and about 20 wt. % of polysulfone, or about 60 wt. % of polystyrene and about 40 wt. %.

In some examples, the polymer comprises high impact polystyrene (HIPS). The polymer can comprise HIPS and a second polymer. The second polymer can be selected from the group consisting of polystyrene, polycarbonate, polypropylene, polyvinylidene fluoride, polycaprolactone, polysulfone, cellulose acetate, cellulose triacetate, polyethersulfone, polyphenylenesulfon, polymethyl methacrylate, acrylonitrile butadiene styrene or mixtures thereof. The composition can comprise HIPS and a second polymer in a wt. % ratio of about 5:95 to about 95:5 (e.g. about 10:90 to about 90:10), more preferably about 20:80 to about 80:20, and even more preferably about 25:75 to about 75:25. HIPS and the second polymer may be present in a wt. % ratio of 30:70, 35:65, 40:60, 45:55, 50:50, 55:45, 60:40, 65:35, 70:30.

The polymer may be present in the composition in an amount of from about 5 wt. % to about 50 wt. %, preferably from about 10 wt. % to about 40 wt. %, from about 15 wt. % to about 30 wt. %.

The wt. % ratio of the non-toxic solvent and the polymer present in the composition may vary depending on the components present and solubility of the polymer thereof. In some examples, the polymer and non-toxic solvent may be present in the composition in a wt. % ratio of about 1: 1 to 10, preferably about 1: 2 to 7, more preferably about 1:3 to 6.

Hollow fibres may be themselves be solid or semi-solid substrates having openings or apertures (pores), which allow components of cell culture media, such as metabolites, nutrients and gases (e.g. oxygen), to be delivered to cells attached to the outer surfaces of the hollow fibres.

Fibres described herein allow the growth of cells on the outer surface of the fibres. Thus the fibres described herein act as a 3-dimensional matrix that allows for the culture and maintenance of cells in a 3-dimensional architecture.

The fibres may have a Young's modulus of at least 1000 Pa. In some examples, the fibres have a Young's modulus from 1000 Pa to 1000000000 Pa. In some examples, the fibres have a Young's modulus between 8000 Pa to about 20000 Pa.

For example, the fibres may have a Young's modulus of around 115 MPa.

For hollow fibres, the lumens of each hollow fibre act as conduits to transport cell culture medium to the cells located on the outer surface of the hollow fibres, as well as transporting waste products from the cells. The density of pores on each hollow fibre may be at least 1 pore/mm2. For example, at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300 pores/mm2.
1. In some examples, the pore density of the hollow fibres may be around 210 pores/mm².

Hollow fibres may be produced by any method, many of which are known in the field. For example, melting spinning, solution spinning, wet spinning, gel spinning, dry-wet spinning, liquid crystal spinning, dispersion spinning, reaction spinning and electrospinning.

In some examples, the hollow fibres may be fibres as described in Luetchford, Kim A., et al. "Next generation in vitro liver model design: Combining a permeable polystyrene membrane with a transdifferentiated cell line." Journal of membrane science 565 (2018): 425-438, which is incorporated herein in its entirety.

### BRIEF DESCRIPTION OF THE DRAWINGS

Examples of the invention are described, by way of example only, with reference to accompanying drawings, in which:
FIG. 1 illustrates an example meat culturing system.
FIG. 2 illustrates a second example meat culturing system.
FIG. 3 illustrates an example meat culturing system with separate process stations for seeding, cell culturing, and harvesting. embodiment.
FIG. 4A illustrates a first example meat culture construct.
FIG. 4B illustrates an end view of the meat culture construct of FIG. 4A.
FIG. 5A illustrates a second example meat culture construct.
FIG. 5B illustrates an end view of the meat culture construct of FIG. 5A.
FIG. 6A illustrates an end view of a third example meat culture construct.
FIG. 6B illustrates an end view of a fourth example meat culture construct.
FIG. 6C illustrates an end view of a fifth example meat culture construct.
FIG. 7A illustrates an example meat culturing system with the meat culture construct of FIG. 4A and FIG. 4B.
FIG. 7B illustrates a further example meat culturing system with the meat culture construct of FIG. 6A.
FIG. 7C illustrates an example meat culturing system with the meat culture construct of FIG. 6B.
FIG. 7D illustrates an example meat culturing system with the meat culture construct of FIG. 6C.
FIG. 8A illustrates an example construct segment of a meat culture construct.
FIG. 8B illustrates a first example meat culture construct with an arrangement of construct segments of FIG. 8A.
FIG. 8C illustrates a second example meat culture construct with an arrangement of construct segments of FIG. 8A.
FIG. 8D illustrates a third example meat culture construct with an arrangement of construct segments of FIG. 8A.
FIG. 9 illustrates a harvesting system.
FIG. 10 illustrates an example meat culture construct with moveable end supports.
FIG. 11 illustrates an example meat culture construct with fluid outlets formed in the end support.
FIG. 12A illustrates an example of using a fluid knife for harvesting.
FIG. 12B illustrates a second example fluid knife for harvesting.
FIG. 13 illustrates an example rotating ball device for harvesting.
FIG. 14A illustrates an example harvesting comb for harvesting.
FIG. 14B illustrates another example harvesting comb for harvesting.
FIG. 14C illustrates another example harvesting comb for harvesting.
FIG. 15 illustrates an example centrifuge system for harvesting.

### DETAILED DESCRIPTION

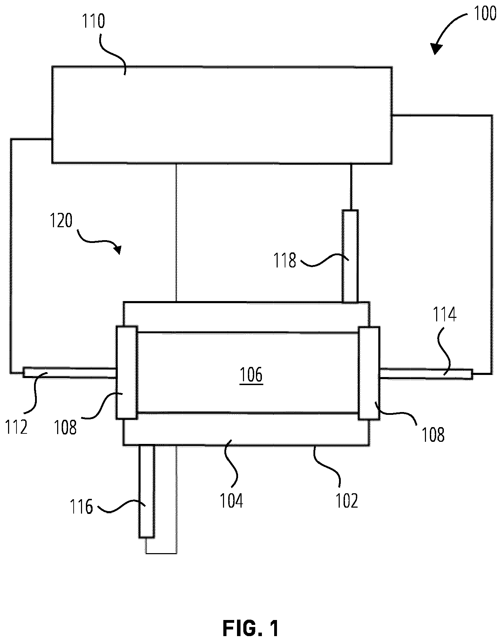

FIG. 1 shows a meat culturing system 100 that includes a meat culture bioreactor module 102. The meat culture bioreactor module 102 includes a housing 104 within which a meat culture construct 106 is received. The meat culture construct 106 comprises end supports 108 and a plurality of hollow fibres with their ends supported in the end supports 108 and extending between the end supports 108 within the housing 104. The hollow fibres have lumina extending through the hollow fibres and open at each end support 108. In this example the end supports 108 are sealed to the housing 104 at each end.

As explained further below, the meat culturing system 100 may be used for seeding, cell culturing, and/or harvesting. One or more of seeding, cell culturing and harvesting may be performed in the meat culturing system 100.

When used for seeding, a cell suspension is flowed into the housing 104 or through the meat culture construct 106 (specifically through the fibres) and cells adhere to external or internal surfaces of the fibres, respectively. When used for cell culturing, cell culture media is flowed into the housing 104 or through the meat culturing system 100 (specifically through the fibres) and cells are cultured on the surface of the fibres. Various harvesting techniques are detailed hereinafter. In examples, the cells may be seeded onto external surfaces of the hollow fibres, or onto internal surfaces of the hollow fibres.

A fluid supply system 110 is connected to the meat culture bioreactor module 102 to provide fluid through the meat culture construct 106 and/or through the housing 104 around the meat culture construct 106.

In particular, the fluid supply system 110 is connected to the end supports 108 at lumen inlet connector 112 and lumen outlet connector 114. The lumen inlet connector 112 and the lumen outlet connector 114 are fluidly connected to the lumina of the hollow fibres in the meat culture construct 106 at the end supports 108.

The fluid supply system 110 is also connected to the housing 104, in particular the volume surrounding the meat culture construct 106 within the housing 104. The fluid supply system 110 is connected to the housing 104 at side inlet connector 116 and side outlet connector 118.

The fluid supply system 110 may include one or more pumps, valves and sensors for controlling the fluid flow rate into/through the housing 104, and into/through the meat culture construct 106.

When the meat culturing system 100 is used for seeding, the fluid supply system 110 can flow a cell suspension through the housing 104 via side inlet connector 116 and side outlet connector 118 such that cells flow over the meat culture construct 106 and adhere to the outer surface of the hollow fibres. Alternatively, the cell suspension may be flowed through lumen inlet connector 112 and lumen outlet connector 114 and through the lumina of the hollow fibres of the meat culture construct 106 to seed cells onto the internal surfaces of the hollow fibres.

Additionally, to assist with cell adhesion, the fluid supply system 110 may create a suction in the lumina of the fibres of the meat culture construct 106 (via lumen inlet connector 112 and/or lumen outlet connector 114) such that cells are pulled onto the outer surfaces of the fibres.

When the meat culturing system 100 is used in cell culturing with cells seeded onto the external surfaces of the hollow fibres the fluid supply system 110 circulates cell culture media through the meat culture construct 106, in particular through the lumina of the hollow fibres. The hollow fibres are permeable to the cell culture media so that circulation of the cell culture media through the hollow fibres feeds the cells on the hollow fibres to support cell culturing.

When the meat culturing system 100 is used in cell culturing with cells seeded onto internal surfaces of the hollow fibres, the fluid supply system 110 circulates cell culture media through the housing 104 (via side inlet connector 116 and side outlet connector 118), around the meat culture construct 106. The fibres are permeable to the cell culture media so that circulation of the cell culture media through the housing 104 feeds the cells within the hollow fibres to support cell culturing

When the meat culturing system 100 is used in cell culturing the meat culturing system 100 may be a perfusion system.

In some examples, the fluid supply system 110 may include means of in-situ sterilisation during operation. For example, the cell culture media may be passed through a UV-light sterilisation step prior to being passed through the bioreactor.

When used in harvesting, the fluid supply system 110 may circulate fluids through the housing 104 and/or meat culture construct 106 to cause detachment of the cells from the hollow fibres. The detached cells can then be removed from the housing 104 and/or meat culture construct 106, for example by being entrained in a flow that carries them out of the meat culturing system 100. Further details of various harvesting techniques are provided hereinafter.

As described above, during seeding, cell culturing and/or harvesting the lumen inlet connector 112, lumen outlet connector 114, side inlet connector 116, and side outlet connector 118 may be used to add, flow or circulate fluid through the meat culture construct 106 (through the fibres) and/or through the housing 104 around and over the meat culture construct 106. Such fluids may include a cultured meat cell suspension (a cell suspension) for seeding, various cell culture media (growth media), buffer solution(s), cleaning fluids, enzyme-containing solutions, antifoam, steam or chemical reagents, for example, for modifying fluid pH.

The lumen inlet connector 112, lumen outlet connector 114, side inlet connector 116, and side outlet connector 118 may each include a threaded male or female type connector, for example, BSP or NPT, or a hygienic specification, for example, Tri-Clamp, Ingold, Luer, Hansen-type, or DIN11851. The lumen inlet connector 112, lumen outlet connector 114, side inlet connector 116, and side outlet connector 118 may each have inner walls that are substantially parallel, curved or tapered. A tapered design for an connector may create a funnel such that the collection of suspended solids, for example cells, are collected more effectively than a straight walled exit port. The side inlet connector 116 and side outlet connector 118 may be arranged relative to the housing 104 such that the are perpendicular to the end supports 108 (as illustrated), or angled with respect to the end supports 108. The lumen inlet connector 112 and lumen outlet connector 114 may be arranged relative to the housing 104 such that the are parallel to the end supports 108 (as illustrated), or angled with respect to the end supports 108.

In some examples, the housing 104 is made of fully welded stainless steel in which the welds are suitably polished and internal surfaces smoothed. That can help to reduce harbouring of microbiological contaminants.

In preferred examples the meat culturing system 100 includes a base unit 120 that holds the meat culture bioreactor module 102. The meat culture bioreactor module 102 may be detachable from the base unit 120 and the fluid supply system 110. The meat culture bioreactor module 102 may be held in a vertical, horizontal, or sloped position.

The housing 104 may be similar to a conventional tubular membrane housing used in filtration applications. As described above, the housing 104 may comprise one or more fluid entry or exit ports enabling fluid to pass in and out of the extra-capillary space within the housing 104, external to the hollow fibres of the meat culture construct 106.

The housing 104 may be substantially similar to a glass or stainless steel bioreactor vessel. The housing 104 may be an upright vessel that is freestanding, or mounted on a frame (base). The housing 104 has an internal height to width aspect ratio of between 0.5 and 5.0. The housing 104 may have a base or top which is conical, dished or flat. The meat culturing system 100, in particular the housing 104, may include a jacket surrounding the side walls of the housing 104, and the jacket may enable temperature maintenance, heating or cooling of the housing 104 by passing fluids such as steam, water or oil through the jacket (in between the jacket and the walls of the housing 104). The meat culturing system 100 may include an incubator, an environmental chamber, or a water bath in which the meat culture bioreactor module 102 may be placed during use. This can help to control temperature and other environmental aspects during meat culturing. The housing 104 may be hermitically sealed by the end supports 108 (and possible additional endplates / head plates), allowing the internal pressure of the housing 104 to be controlled, for example elevated. This can also maintain the sterility of the internal space of the housing 104 to avoid microbiological or other contaminant ingress into the housing 104 during operation. The housing 104, in particular the side walls and/or the end supports 108 and/or any additional endplate/headplate, may include one or more ports that allow sensors and/or gauges to be connected. Sensors and/or gauges may be provided to detect pressure, temperature, . Sensors may include one or more of pressure sensors, temperature sensors (e.g., thermowells), pH sensors, foam sensors, level sensors (e.g., point level switches), dissolved oxygen (dO) sensors, capacitance sensors, gas analysers, or optical density sensors. The housing 104, in particular the side walls and/or the end supports 108 and/or any additional endplate/headplate, may include one or more ports that allow equipment to be connected. For example, fixed or rotating spray heads (e.g., spray balls) for cleaning, bursting discs, pressure relief valves, overflow pipes, condensers, agitator shafts, sight glasses, lights, addition tubes, or dip tubes.

In some examples, the housing 104 may be a modified bioreactor enabling retro-fitting of the meat culture construct 106.

The end supports 108 may comprise a sealing material, for example a resin, glue, or potting material. The sealing material can be introduced between the plurality of fibres in a fluid form, for example using a centrifuge, and then cured to form the end support 108. After curing an end of the end support 108 may be cut off to expose ends of the fibres and their lumina. In some examples, the end supports 108 comprise a cup (e.g., a tube) within which the fibres are sealed.

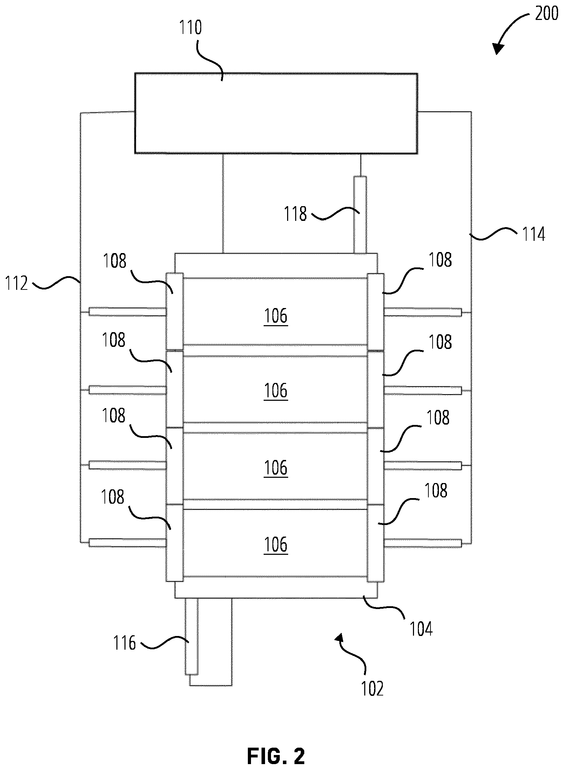

FIG. 2 shows a meat culturing system 200 that is similar to the meat culturing system 100 of FIG. 1 except that a plurality of meat culture constructs 106 are received in the housing 104. A single fluid supply system 110 is connected to the housing 104 at side inlet connector 116 and side outlet connector 118 as in FIG. 1. In other examples, multiple side inlet connectors 116 and side outlet connectors 118 may be provided. The fluid supply system 110 is also connected to each of the meat culture constructs 106 via lumen inlet connectors 112 and lumen outlet connectors 114.

The housing 104 and connectors may be as described above with respect to FIG. 1, but larger to accommodate multiple meat culture constructs 106.

Each of the meat culture constructs 106 may be independently removable from the housing 104. Different meat culture constructs 106 may be added to, and removed from, the meat culturing system 100 at different times. The meat culture constructs 106 may be consumable assemblies that are provided to the meat culturing system 200. In some examples the meat culturing system 200 may be provided at a manufacturing site, and the meat culture constructs 106 may be provided (i.e., shipped) to the manufacturing site for use in the meat culturing system 200.

The meat culturing system 200 of FIG. 2 works in the same way as the meat culturing system 100 of FIG. 1. Providing multiple meat culture constructs 106 in a single housing 104 increases the production capacity of the meat culturing system 200. In addition, meat culture constructs 106 may be added and removed at different times.

The meat culture constructs 106 may be removable from the housing 104. The meat culture construct 106 may be removable from a top of the housing 104 (i.e., in a vertically upwards direction) allowing fluid to remain present in the housing 104 as one or more meat culture constructs 106 are removed.

As illustrated, in the example of FIG. 2 a single fluid supply system 110 can provide process fluids to multiple meat culture constructs 106. Depending on the process being performed (e.g., seeding, cell culturing, harvesting, cleaning), the process fluids may include a cell suspension, cell culture media (growth media) and/or other process fluids.

The fluid supply system 110 may include one or more pumps, valves and sensors for controlling the fluid flow rate to the housing 104 and each of the meat culture constructs 106. The fluid supply system 110 may be adapted to provide the same or different fluids to different meat culture constructs 106. The fluid supply system 110 may be configured (e.g., with various valves and pumps) to independently control the fluid flow rate for each of the meat culture constructs 106. In examples, the fluid supply system 110 comprises one or more "ring-main" conduits for one or more process fluids, and valves and/or pumps to control flow of the process fluids from the ring-main(s) into the housing 104 and/or meat culture constructs 106.

In examples, the meat culturing system 200, and in particular the fluid supply system 110, may be configured to allow different meat culture constructs 106 to be processed differently within the same housing 104. For example, some of the meat culture constructs 106 may be processed for seeding, some for cell culturing, and some for harvesting. Alternatively, all of the meat culture constructs 106 are subject to the same process.

The meat culturing system 100 and meat culturing system 200 are for culturing meat. In particular, the cells seeded onto the meat culture constructs 106 may include muscle cells and/or muscle cell precursors. Muscle cells include those cells making up contractile tissue of animals or cells that can differentiate into muscle cells. Muscle cells are derived from the mesodermal layer of embryonic germ cells. Mature muscle cells contain contractile filaments that move past each other and change the size of the cell. They are classified as skeletal, cardiac, or smooth muscles. As used herein, the term "cells that can differentiate into muscle cells" and "muscle cell precursors" refers to stem cells (e.g., induced pluripotent stem cells (iPSC) or embryonic stem cells (ESC)), muscle progenitor cells that can differentiate into muscle cells (e.g. mature muscle cells), and/or myosatellite cells.

Muscle cells may include those cells normally found in muscle tissue, including smooth muscle cells, cardiac muscle cells, skeletal muscle cells (e.g., muscle fibres or myocytes, myoblasts, myotubes, etc.), and any combination thereof. Muscle cells may include myoblasts, myotubes, myofibrils, and/or satellite cells.

The cells may further include adipose or fat cells or fat precursor cells. Such fat cells may include any cell or group of cells composed in a fat tissue, including, for example, lipocytes, adipocytes, adipocyte precursors including, pre-adipocytes, adipogenic stem cells, and mesenchymal stem cells.

The cells used in the meat culturing system 100 and meat culturing system 200 may comprise more than one type of cell in various combinations - a cell co-culture.

The cells may be derived from any source animal. As the bioreactor systems described herein may be for use in making comestible products, the cells may not be derived from a human. In some examples, the cells may be derived from bovine, ovine, equine, porcine, caprine, avian, fish, insect, crustaceans, cephalopod, mollusc and/or camelid animals. Preferably the cells may be derived from a bovine, porcine, avian and/or ovine animal. For example, the cells may be derived from a cow, pig, chicken, fish, squid, insect, oyster and/or sheep.

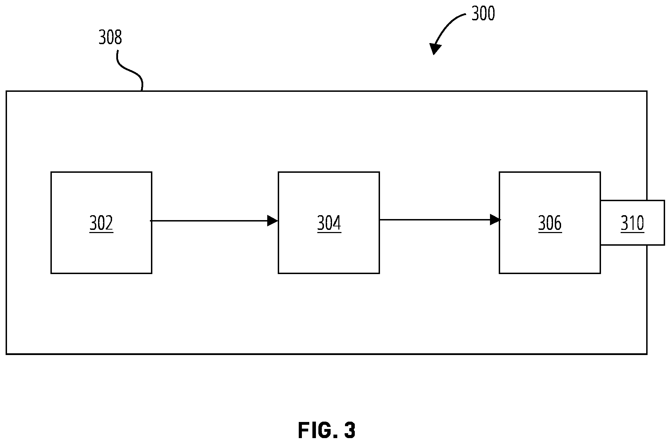

FIG. 3 shows a meat culturing system 300 with three separate stations. In the meat culturing system 300 the meat culture constructs may be seeded at a seeding station 302, transferred to a cell culturing station 304 for culturing, and then transferred to a harvesting station 306 for harvesting. The meat culture constructs may be transferred between the seeding station 302, cell culturing station 304, and harvesting station 306 manually or automatically, for example by a conveyor or robotic system.

Each of the seeding station 302, cell culturing station 304, and harvesting station 306 may be the same or similar to the meat culturing system 100 or the meat culturing system 200 described above, with a housing 104 within which one or more meat culture constructs 106 are received and a fluid supply system 110 to supply fluid to the housing 104 and/or meat culture constructs 106.

As illustrated, the seeding station 302, cell culturing station 304 and harvesting station 306 may be provided within an overall system housing 308. The system housing 308 may define a sterile area within which the seeding, culturing and harvesting processes are conducted. Each of the seeding station 302, cell culturing station 304 and harvesting station 306 may include its own housing for holding the meat culture constructs 106 and any fluid (e.g., cell suspension or cell culture media).

At the seeding station 302 a cell suspension is provided, either in the housing 104 or through the hollow fibres of the meat culture constructs 106, such that cells adhere to the meat culture constructs 106.

At the cell culturing station 304 cell culture media is provided to promote cell culturing.

At the harvesting station 306 the cells are harvested. Harvesting comprises detaching the cells from the meat culture construct 106 and removing and/or collecting the detached cells. In particular, harvesting includes two stages: (1) detachment of cells from the meat culture construct 106 (particularly the hollow fibres), and (2) removal of the cells to different location, for example to be cleaned, processed, and collected. Various example harvesting techniques are described further hereinafter.

In examples, the meat culturing system 300 may include a cell collection station 310 where cells are collected after being harvested. The cell collection station 310 may be a part of the harvesting station 306, or separate. The cell collection station 310 is preferably within the sterile space, in particular within the system housing 308. In some examples, the cell collection station 310 extends beyond (e.g., through) the system housing 308, allowing cells to be collected from outside the system housing 308.

At the harvesting station 306 detached cells can be suspended or entrained within fluid within the housing containing the meat culture construct. The cell collection station 310 can receive the fluid with entrained cells. At the cell collection station 310 the fluid containing the detached cells can then be concentrated by any suitable means including but not limited to, filtration, sedimentation, centrifugation, flocculation including foam flotation and drying. The fluid containing the entrained cells can be drained via suitable ports so as to allow concentration elsewhere. A housing of the cell collection station 310 can be shaped so as to allow easy collection of fluid and sedimentation, as well as easy cleaning. The concentrated and recovered cells can be subjected to further processes and conditioning, including washing/purification and heating/cooling. Such further processes may occur within, or outside of, the system housing 308.

In examples, the detached cells may include intact cells or non-intact cells (e.g., protein isolates) and other cultured products, including for example exogenous proteins including extracellular matrix.

In some examples, the meat culturing system 300 may further comprise a cleaning station where the meat culture constructs are cleaned and sterilised after harvesting. The cleaning station may be located within the system housing 308. After cleaning, the meat culture constructs can be returned to the seeding station 302 and re-used.

In other examples, the meat culture constructs may be cleaned within the harvesting station 306, for example after harvesting cells. Cleaning may clean the meat culture constructs and/or other parts of the meat culturing system 300, in particular the housings, fluid supply system components (pipes, tubes, connectors, pumps, valves etc...) and other components.

The meat culture construct(s), housing(s) and other components are preferably sterilized. Sterilisation may be carried out by various means including steam, extended exposure to temperatures between 60 degrees Celsius and 100 degrees Celsius, contact with peracetic acid and other bleaching species (e.g., hydrogen peroxide, sodium hypochlorite), and/or exposure to UV-light. For example, the external surfaces of the housing and bioreactor can be sterilised by exposure to high intensity UV-light or fogging with peracetic acid mists. Such techniques are used in healthcare applications.

The meat culturing systems described herein are capable of aseptic operation. Materials of construction for any vessels, housings, fittings, sensors, pumps and pipes are suitable and appropriate for aseptic use and food contact. This includes ease of disassembly for cleaning, including within a sterile environment and/or in a non-sterile environment.

The various components of the meat culturing systems, in particular the housings 104, 308, fittings, sensors, pumps and pipes, may be made from stainless steel, for example one or more of stainless steel grades; 304, 304L, 416, 316L, Duplex; 2205, 2207, 2507, Hastelloys; C-22, C-22HS,C-263, C-276

In other examples, one or more of the various components may be made from one or more of glass, borosilicate glass, or plastics, for example Polysulfone, Acrylonitrile butadiene styrene (ABS), Nylon 6, Nylon 6-6, Polyamide (PA), Polybutylene terephthalate (PBT), Polycarbonate (PC), Polyetheretherketone (PEEK), Polyetherimide (PEI), Polyetherketoneketone (PEKK), Polyetherketones (PEK), Polyketone (PK), Polyethylene terephthalate (PET), Polyoxymethylene plastic (POM / Acetal), Polyphenylene sulfide (PPS), Polyphenylene oxide (PPO), Polypropylene (PP), Polystyrene (PS), Polysulphone (PSU), Polytetrafluoroethylene (PTFE / Teflon), Poly(methyl methacrylate) (PMMA) or Polyvinyl Chloride (PVC).

Specifically, any part of the meat culturing systems that is not the hollow fibres may be made from one or more of the above materials.

The housings and other parts of the meat culturing systems are designed to facilitate and simplify sterile operations. For example, the meat culturing systems are designed for sterile operation by an operator in an external, non-sterile area. The meat culturing systems, or parts thereof, can be contained within a sterile environment or room. This may be defined by the system housing 308. In examples, it could be advantageous to be able to remove the meat culture constructs from the meat culturing systems within a sterile area, for example within the system housing 308.

Any sterile area can contain one or more meat culturing systems. The meat culturing systems can be mounted in any appropriate manner. For example, the meat culturing systems could be stacked on top of each other within a sterile area to form an array of meat culturing systems. The meat culturing systems can be mounted so as to allow easy attachment and detachment.

The sterile areas or spaces (e.g., the system housing 308) can be at the same temperature as non-sterile areas, or can be at a different temperature to the surrounding areas. For example, the space surrounding the meat culturing system (in particular the housing 104) could be at a low temperature so as to maintain liquid quality in storage vessels whilst the actual housing 104 is maintained at a higher temperature for cell growth. The sterile areas can be supplied with heating means, for example to heat the housings 104 and meat culture constructs 106 as shown in FIG. 1 and FIG. 2. The sterile areas can also be supplied with sensors and other means of detecting and controlling conditions within the sterile and non-sterile areas.

It is advantageous if the size of any sterile area is kept as small as possible to reduce the overall size and due to costs associated with air flows and other services to maintain sterility. The dimensions of a sterile area (e.g., the system housing 308) can be such that it closely matches the external dimensions of the meat culturing system and its components whilst allowing space for the required unit operations, such as removal of modules.

As described above, in some examples the meat culturing system 300 can be designed such that the different unit operations can happen in the same space or can happen in different stations, such as stations for seeding, cell culturing (cell growth), and harvesting. In examples, the meat culture constructs 106 could be removed, either manually or by robot, from one area/station, such as the cell culturing station 304 or the seeding station 302, to another area/station so as to allow another unit operation to take place, such as harvesting or cleaning.

The meat culturing system is provided with appropriate utilities, such as electricity, purified air, etc and an appropriate control system capable of running and integrating the various unit operations. The meat culturing system has means for controlling the composition of the fluids within and surrounding the meat culture constructs, for example the level of dissolved oxygen and dissolved growth media and metabolites. The meat culturing system comprises storage and feeding means as well as means of purifying and recycling media as well as oxygenating the fluids.

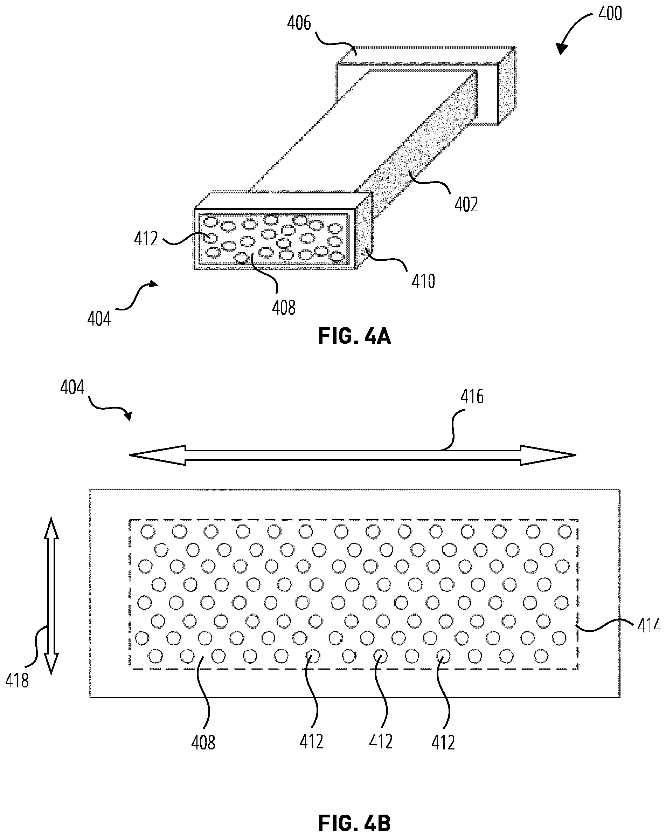

FIG. 4A and FIG. 4B show a first example meat culture construct 400 that may be the meat culture construct(s) 106 of FIG. 1 and FIG. 2.

As shown, the meat culture construct 400 includes a fibre bundle 402 extending between a first end support 404 and a second end support 406. The first end support 404 and the second end support 406 are the same. In this example the first end support 404 includes a potting material 408 in which ends of the fibres 412 of the fibre bundle 402 are embedded. The fibres 412 are hollow fibres, and lumina of the fibres 412 are open at the first end support 404. In this example the first end support 404 further includes an end support side 410. The end support side 410 may be a side surface of the potting material 408, or it may comprise a frame or cup that holds the potting material 408. The end support side 410 may be formed by a tubular member within which the ends of the fibres 412 are potted.

When the meat culture construct 400 is used in the meat culturing system 100 described above the end support side 410 may seal against the housing 104, for example against a part of a side wall or endplate of the housing 104. When the meat culture construct 400 is used in the meat culturing system 200 described above the end support side 410 may seal against the housing 104 (e.g., against a side wall and/or endplate of the housing 104) and/or against an adjacent meat culture construct 400. In this way, multiple meat culture constructs 400 can be provided in the housing 104 and they can seal against each other. The end support side 410 may include a seal, such as a gasket, to create the sealing.

As shown in the end view of FIG. 4B, the fibres 412 are embedded in the first end support 404 within an envelope 414. That is, all the fibres 412 are arranged within the envelope 414 in the plane of the end of the first end support 404.

Within the envelope 414 the fibres 412 are arranged in a fibre distribution. The fibres 412 are spaced apart, such spaces are formed between the fibres 412 where they extend between the first end support 404 and the second end support 406. The fibre distribution may be regular or irregular, and may have a minimum and/or maximum fibre spacing.

In this example the envelope 414 and the first end support 404 are rectangular. The envelope 414 has a first dimension 416 and a second dimension 418. The first dimension 416 is greater than the second dimension 418. In the illustrated example, a ratio of the first dimension 416 to the second dimension 418 is about 3:1. In various examples the ratio may be at least 2:1, for example at least 2.5:1, for example at least 3:1, for example at least 3.5:1, for example at least 4:1, for example at least 5:1, for example at least 10:1, for example at least 20:1.

In larger scale examples the ratio may be even higher, for example at least 50:1, for example at least 100:1, for example at least 200:1, for example at least 300: 1, for example at least 500:1, for example at least 500:1. In examples, the ratio may be at most 500:1, for example at most 750:1, for example at most 1000:1. In preferred examples the ratio may be between 2:1 and 1000:1, for example between 2:1 and 500:1, for example between 2:1 and 100:1, for example between 2:1 and 50:1. In examples, the ratio may be between 3:1 and 500:1, for example between 3:1 and 100:1, for example between 3:1 and 50:1.

In this way, the envelope 414 has high aspect ratio - one dimension is greater than the other dimension. This means that the fibre bundle 402 has a small thickness compared to its width relative to the direction of fluid flow.

The first dimension 416 is the dimension of the fibre bundle 402 against which fluid flows during use (seeding, culturing and/or harvesting).

As explained in detail below, this ratio of the first dimension 416 to the second dimension 418 improves the seeding, culturing and harvesting processes performed using the meat culture construct 400.

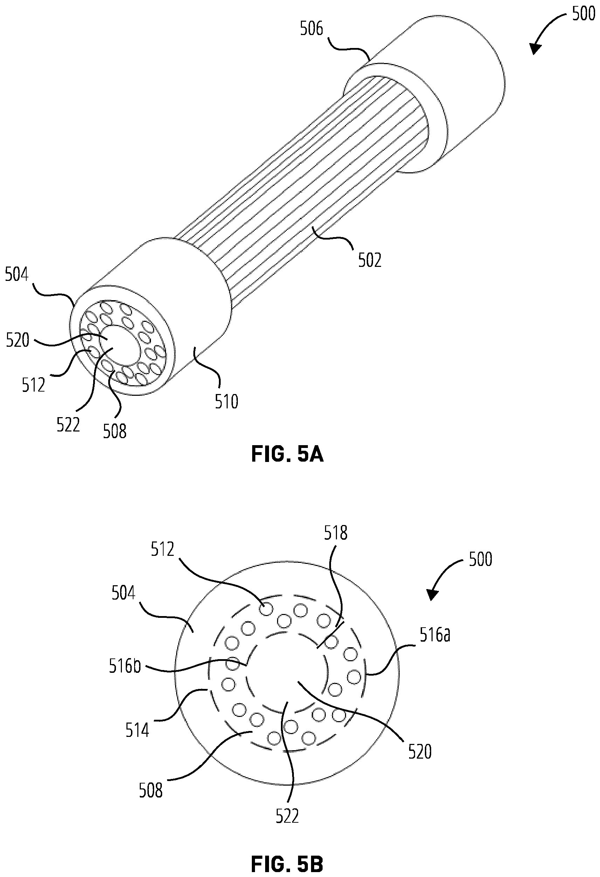

FIG. 5A and FIG. 5B show a second example meat culture construct 500 that may be the meat culture construct(s) 106 of FIG. 1 and FIG. 2.

As shown, the meat culture construct 500 includes a fibre bundle 502 extending between a first end support 504 and a second end support 506. The first end support 504 and the second end support 506 are the same. In this example the first end support 504 includes a potting material 508 in which ends of the fibres fibre 512 of the fibre bundle 502 are embedded. The fibres fibre 512 are hollow fibres, and lumina of the fibres 512 are open at the first end support 504. In this example the first end support 504 includes an end support side 510. The end support side 510 may be a side surface of the potting material 508, or it may comprise a frame or cup that holds the potting material 508.

When the meat culture construct 500 is used in the meat culturing system 100 described with reference to FIG. 1 the end support side 510 may seal against the housing 104, for example in a circular hole of the housing 104. When the meat culture construct 500 is used in the meat culturing system 200 described with reference to FIG. 2 the housing 104 may include a plurality of holes to receive a plurality of meat culture constructs 500. In this way, one or more meat culture constructs meat culture construct 500 can be provided in the housing 104 and they can seal against the housing. The end support side 510 may include a seal, such as a gasket, to create a sealing with the housing 104.

As shown in the end view of FIG. 5B, the fibres 512 are embedded in the first end support 504 within an envelope 514. That is, all the fibres 512 are arranged within the envelope 514 in the plane of the end of the first end support 504.

Within the envelope 514 the fibres 512 are arranged in a fibre distribution. The fibres 512 are spaced apart, such spaces are formed between the fibres 512 where they extend between the first end support 504 and the second end support 506. The fibre distribution may be regular or irregular, and may have a minimum and/or maximum fibre spacing.

In this example the first end support 504 is circular and the envelope 514 is formed as an annular region about a central channel 520. The central channel 520 extends between the first end support 504 and the second end support 506.

The meat culture construct 500 may be made by potting the fibres 512 in the annular shape illustrated. Alternatively, the fibres 512 may be potted in a 2D array and then rolled into the annular shape illustrated, held in form by a cup or similar that provides the end support side 510.

The central channel 520 may be used to pass fluid through the centre of the envelope 514 in the meat culturing system 100 or meat culturing system 200. In particular, one or both of the side inlet connector 116 and the side outlet connector 118 may connect with the central channel 520, for example at opposite ends of the central channel 520. Fluid may be passed into the central channel 520 such that it passes through the fibre bundle 502, thereby passing over the fibres 512. In other examples, fluid may be sucked from the central channel 520, reversing the direction of fluid flow through the fibre bundle 502. The flow direction may be changed (reversed) during use.

A tube, for example a distribution tube 522, may extend through the central channel 520. The distribution tube 522 may be permeable or perforated, for example having a plurality of openings. The distribution tube 522 may act to support the fibres 512, holding them within the envelope 514. The distribution tube 522 may be removable from the central channel 520 or fixed therein.

The distribution tube 522 may include a plurality of openings along the length of the tube, each opening connecting the central channel 520 to the volume surrounding the fibres 512. In such examples a distribution of the openings may be arranged to control fluid flow into / out of the envelope 514 to / from the central channel 520. For example, there may be more and/or larger openings in the end of the distribution tube 522 closer to the second end support 506 relative to the first end support 404 such that fluid flowing into the central channel 520 from the first end support 404 is more evenly distributed along the length of the meat culture construct 500.

The central channel 520 and/or distribution tube 522 may additionally or alternatively be used to house a sensor or probe, for example a dissolved oxygen (dO) sensor, a pH sensor, or a capacitance sensor.

The central channel 520 and/or distribution tube 522 may additionally or alternatively be used to house a heating device for heating the meat culture construct 400.

The central channel 520 and/or distribution tube 522 may additionally or alternatively be used for cleaning the meat culture construct 400. For example, a rotating spray ball, jet or spray head (for cleaning fluids and/or steam), or the like, may be passed into the central channel 520 or distribution tube 522 for cleaning the meat culture construct 500.

The central channel 520 and/or distribution tube 522 may additionally or alternatively be used in various harvesting techniques as described hereinafter.

The envelope 514 has a first dimension 516a, 516b and a second dimension 518. The first dimension 516a, 516b is greater than the second dimension 518. The first dimension 516a may be the circumference of the outer boundary of the envelope 514 or the first dimension 516b may be the circumference of the inner boundary of the envelope 514, depending on the direction of fluid flow (from within the central channel 520, through the fibre bundle 502 to the outside, or from the outside, through the fibre bundle 502 into the central channel 520). In both cases, the first dimension 516a, 516b is the dimension of the fibre bundle 502 against which fluid flows during use (seeding, culturing and/or harvesting). The first dimension 516a, 516b is the dimension of the fibre bundle 502 in a direction perpendicular to the fluid flow direction.

The second dimension 518 is the thickness of the fibre bundle 502 in the direction of fluid flow during use (in particular during seeding, culturing and harvesting).

In the illustrated example, a ratio of the first dimension 516a, 516b to the second dimension 518 is about 4:1. In various examples the ratio may be at least 2:1, for example at least 2.5:1, for example at least 3:1, for example at least 3.5:1, for example at least 4:1, for example at least 5:1, for example at least 10:1, for example at least 20:1. For larger scale meat culture constructs 500 the ratio may be higher, for example at least 50:1, for example at least 100:1, for example at least 200:1, for example at least 300:1, for example at least 500:1, for example at least 500:1. In examples, the ratio may be at most 500:1, for example at most 750:1, for example at most 1000:1. In preferred examples the ratio may be between 2:1 and 1000: 1, for example between 2:1 and 500:1, for example between 2:1 and 100:1, for example between 2:1 and 50:1. In examples, the ratio may be between 3:1 and 500:1, for example between 3:1 and 100:1, for example between 3:1 and 50:1.

In this way, the envelope 514 effectively has a high aspect ratio - one dimension is greater than the other dimension with respect to the fluid flow direction during use. This means that the fibre bundle 502 has a small thickness compared to its width relative to the direction of fluid flow.

As explained in detail below, this ratio of the first dimension 516a, 516b to the second dimension 518 improves the seeding, culturing and harvesting using the meat culture construct 500.

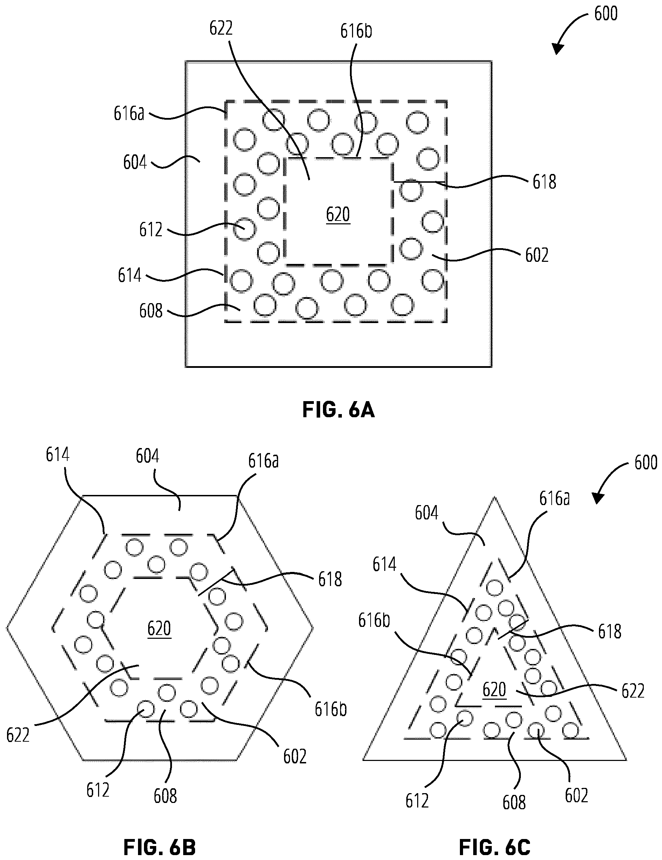

The examples of FIG. 6A, FIG. 6B and FIG. 6C are similar to that of FIG. 5A and FIG. 5B, but with different shapes. The example of FIG. 6A is square, the example of FIG. 6B is hexagonal, and the example of FIG. 6C is triangular.

As with the example of FIG. 5A and FIG. 5B, the end supports 604 of one or more meat culture constructs 600 can be received and sealed in the housing 104 of the meat culturing system 100 or meat culturing system 200.

In each example meat culture construct 600 of FIG. 6A to FIG. 6C the meat culture construct 600 includes a fibre bundle 602 embedded within an end support 604 (second end support not illustrated). A potting material 608 is provided in the end support 604, in which a plurality fibres 612 are embedded. The fibres 612 are arranged in a fibre distribution within an envelope 614 defined in the plane of the end support 604. A central channel 620 is formed through the envelope 614. The envelope 614 has a first dimension 616a, 616b and a second dimension 618. The central channel 620 may comprise a distribution tube 622, as described above with reference to FIG. 5A and FIG. 5B. The central channel 620 and/or distribution tube 622 may be used in the same manner as described above with reference to FIG. 5A and FIG. 5B.

The meat culture constructs 600 may be made by potting the fibres 612 in the annular shape illustrated. Alternatively, the fibres 612 may be potted in a 2D array and then rolled or folded into the annular shapes illustrated.

The first dimension 616a may be the outer length of the outer boundary of the envelope 614, or the first dimension 616b may be the length of the inner boundary of the envelope 614. The first dimension 616a, 616b is the dimension of the fibre bundle 602 in a direction perpendicular to the fluid flow direction.

The second dimension 618 is the thickness of the fibre bundle 602 in the direction of fluid flow during use (in particular during seeding, culturing and harvesting). The second dimension 618 is the distance between the inner and outer boundaries of the envelope 614. The second dimension 618 is therefore the thickness of the fibre bundle 602 in the direction of fluid flow during use (in particular during seeding, culturing and harvesting).

In the illustrated example, a ratio of the first dimension 616a, 616b to the second dimension 618 is about 4:1. In various examples the ratio may be at least 2:1, for example at least 2.5:1, for example at least 3:1, for example at least 3.5:1, for example at least 4:1, for example at least 5:1, for example at least 10:1, for example at least 20:1. For larger scale meat culture constructs 500 the ratio may be higher, for example at least 50:1, for example at least 100:1, for example at least 200:1, for example at least 300:1, for example at least 500:1, for example at least 500:1. In examples, the ratio may be at most 1000: 1.

In this way, the envelope 614 effectively has a high aspect ratio - one dimension is greater than the other dimension with respect to the direction of fluid flow during use. This means that the fibre bundle 602 has a small thickness compared to its width relative to the direction of fluid flow.

As explained in detail below, this ratio of the first dimension 616a, 616b to the second dimension 618 improves the seeding, culturing and harvesting using the meat culture construct 600.

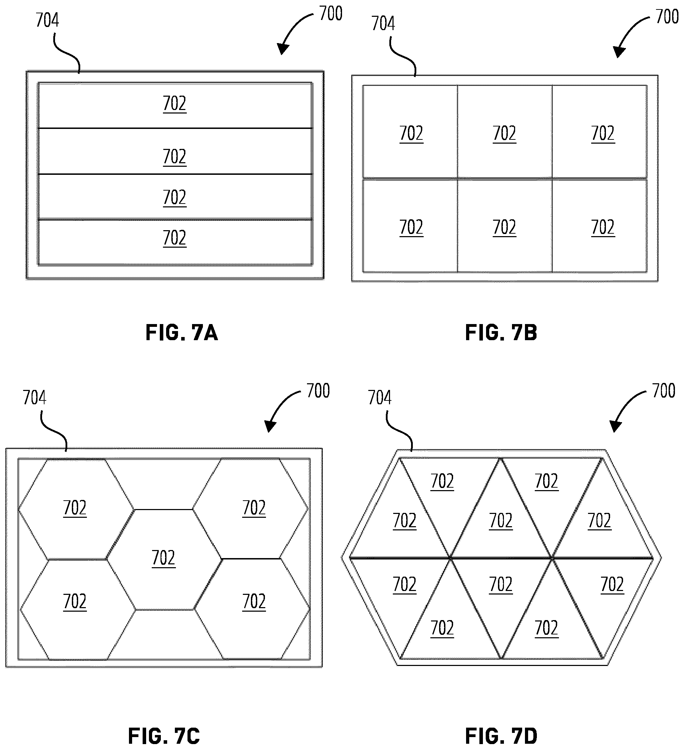

FIG. 7A to FIG. 7D show various arrangements of meat culture constructs 702 of different shapes within a housing 704 of a meat culturing system 700. The housing 704 may be the housing 104 of the meat culturing system 200 described with reference to FIG. 2. FIG. 7A to FIG. 7D show top (plan) views of the meat culturing system 700, with multiple meat culture constructs 702 arranged within the housing 704. As described with reference to FIG. 2, a fluid supply system 110 can be connected to the housing 704 and to the meat culture constructs 702 to circulate fluids during use (in particular during seeding, cell culturing and harvesting).

In the example of FIG. 7A the meat culture constructs 702 are as described with reference to FIG. 4A and FIG. 4B, with a rectangular shape. The meat culture constructs 702 each comprise a rectangular end support. A plurality of meat culture constructs 702 are arranged with their longer dimensions in abutment within the housing 704. Each meat culture construct 702 can be added to / removed from the housing 704 individually. The meat culture constructs 702 may seal against each other. The housing 704 may comprise a frame to hold the end supports of the meat culture construct 702.

In the example of FIG. 7B the meat culture constructs 702 are as described with reference to FIG. 6A, with a square shape. The meat culture constructs 702 each comprise a square end support. A plurality of meat culture constructs 702 are arranged in two abutting rows within the housing 704. Each meat culture construct 702 can be added to / removed from the housing 704 individually. The meat culture constructs 702 may seal against each other. The housing 704 may comprise a frame to hold the end supports of the meat culture construct 702.

In the example of FIG. 7C the meat culture constructs 702 are as described with reference to FIG. 6B, with a hexagonal shape. The meat culture constructs 702 each comprise a hexagonal end support. A plurality of meat culture constructs 702 are arranged in an abutting honeycomb arrangement within the housing 704. Each meat culture construct 702 can be added to / removed from the housing 704 individually. The meat culture constructs 702 may seal against each other. The housing 704 may comprise a frame to hold the end supports of the meat culture construct 702.

In the example of FIG. 7D the meat culture constructs 702 are as described with reference to FIG. 6C, with a triangular shape. The meat culture constructs 702 each comprise a triangular end support. A plurality of meat culture constructs 702 are arranged in an abutting tessellated arrangement within the housing 704. Each meat culture construct 702 can be added to / removed from the housing 704 individually. The meat culture constructs 702 may seal against each other. The housing 704 may comprise a frame to hold the end supports of the meat culture construct 702.

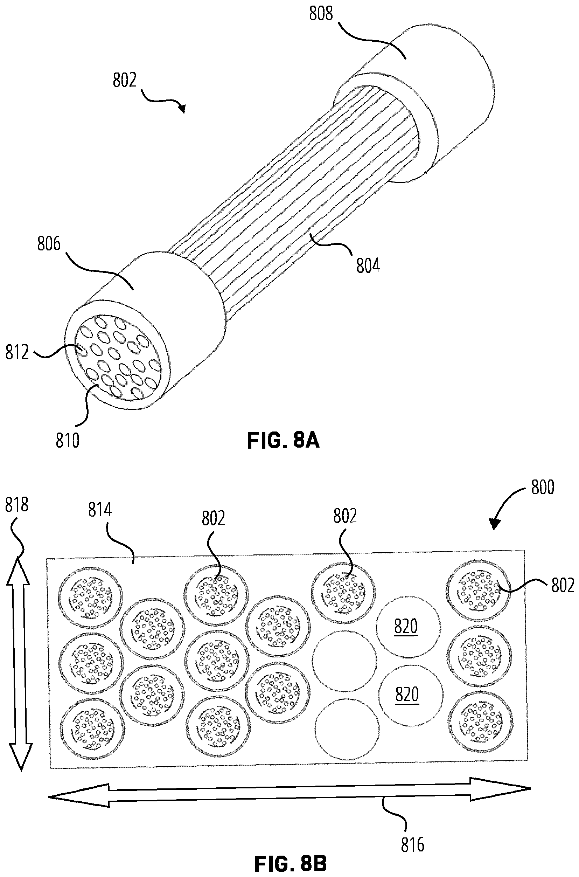

FIG. 8A and FIG. 8B illustrate an example meat culture construct 800 that is formed of a plurality of construct segments 802. FIG. 8A shows a single construct segment 802, and FIG. 8B shows a plurality of construct segments 802 combined to form the meat culture construct 800.

As shown in FIG. 8A, the construct segment 802 comprises a fibre bundle 804 with a first end support segment 806 and a second end support segment 808. The fibre bundle 804 is formed of a plurality of fibres 812 with their ends embedded in potting material 810 within the first end support segment 806 and second end support segment 808.

As shown in FIG. 8B, the meat culture construct 800 is constructed by assembling a plurality of the construct segments 802 illustrated in FIG. 8A.

In the illustrated example the meat culture construct 800 comprises an end support 814. There may be another end support 814 at the other end of the meat culture construct 800, as with other examples. A plurality of construct segments 802 are assembled with the end support 814 to form the meat culture construct 800.

In some examples the first end support segments 806 (and optionally also the second end support segments 808) may be potted together with potting material to form the end support 814.

In other examples, the end support 814 may comprise a plate with holes 820 into which the first end support segments 806 (and optionally also the second end support segments 808) can be mounted. For example, the first end support segments 806 may be push-fit or interreference fit with the holes 820, or the holes 820 may comprise a thread that cuts or grips the first end support segments 806 as they are twisted into the holes 820. In examples, the plate (end support 814) may comprise a rigid material, such as metal (stainless steel) or a rigid polymer, or a soft or elastic material such as silicone.

As shown in FIG. 8B, once assembled the meat culture construct 800 has a first dimension 816 and a second dimension 818 in the same manner as described with reference to FIG. 4B.

In this way, the meat culture construct 800 is constructed of a plurality of construct segment 802. Advantageously, larger meat culture constructs 800 can be manufactured in this way using a conventionally sized centrifuge to form the construct segments 802, which are then assembled together. This may increase the size of the meat culture constructs 800 and increase the production capacity of the meat culturing system. Additionally, it may be simpler and more accurate to form a plurality of construct segments 802 that the larger meat culture constructs described above, particularly for larger scales as a standard size centrifuge can be used to pot the fibres 812.

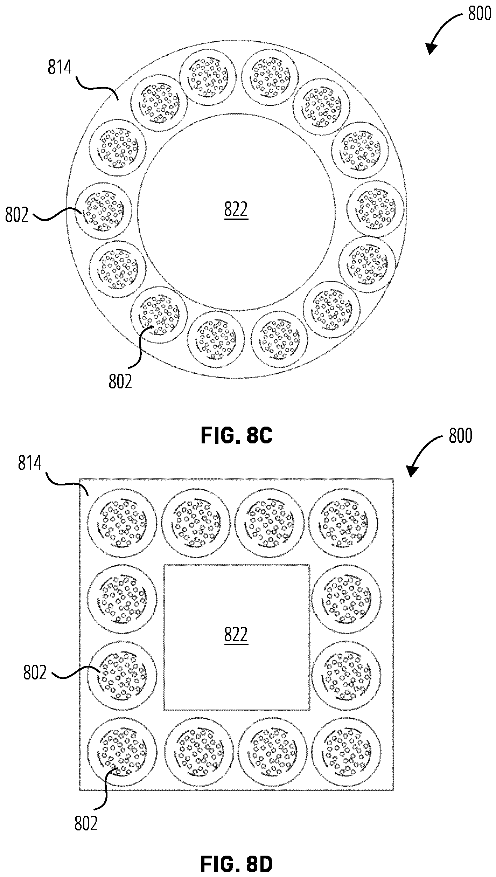

The example of FIG. 8C is similar to that FIG. 8A and FIG. 8B except that the meat culture construct 800 is circular, and the envelope is annular with a central channel 822 like the meat culture construct 500 of FIG. 5A and FIG. 5B. A plurality of construct segments 802 can be assembled and joined to each other at the end support 814 to form the meat culture construct 800.

The example of FIG. 8D is similar to that FIG. 8A and FIG. 8B except that the meat culture construct 800 is square, and the envelope has a central channel 822 like the meat culture construct 500 of FIG. 6A. A plurality of construct segments 802 can be assembled and joined to each other at the end support 814 to form the meat culture construct 800.

In other examples, the meat culture construct 800 may be hexagonal, like that of FIG. 6B, or triangular, like that of FIG. 6C.

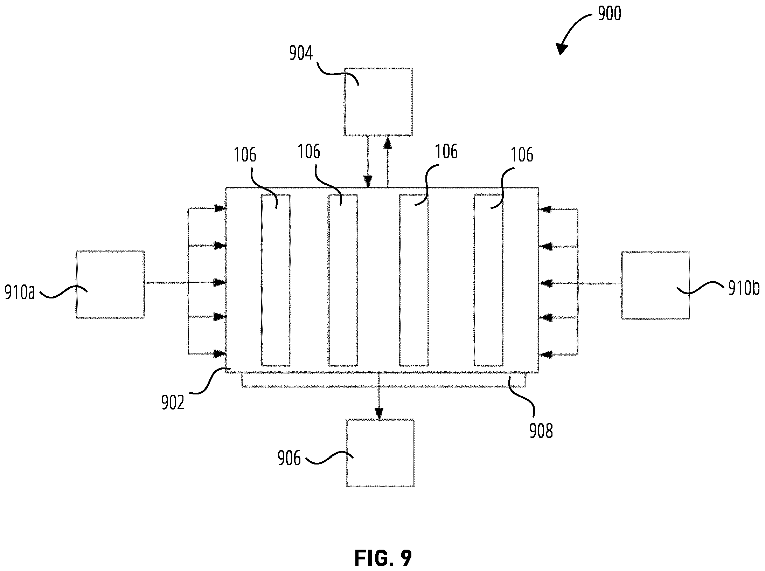

FIG. 9 illustrates a harvesting system 900. The harvesting system 900 may be provided as the harvesting station 306 described with reference to FIG. 3. The harvesting system 900 may be structurally the same, or similar, to the meat culturing system 100 described with reference to FIG. 1 or the meat culturing system 200 described with reference to FIG. 2. In particular, the harvesting system 900 includes a housing 902 within one or more meat culture constructs 106 are received. In the illustrated example, a plurality of meat culture constructs 106 are received in the housing 902. One or more fluid supply systems (described below) may be provided to flow fluid into/through the housing 902 and/or the meat culture constructs 106.

As described with reference to FIG. 3, the harvesting system 900 receives the meat culture constructs 106 from a cell culturing station 304. Alternatively, one or more of the meat culture constructs 106 may be transitioned from a cell culturing process to a harvesting process within the same system, for example by changing the type of fluid being provided and/or performing different processes on the meat culture constructs 106.

The meat culture constructs 106 may be any of the meat culture constructs 106, 400, 500, 600, 702, 800 described above.

As illustrated, the meat culture constructs 106 (with cultured cells on their outer surfaces, ready to be harvested) are placed in the housing 902 of the harvesting system 900. The meat culture constructs 106 are spaced apart within the housing 902. This creates space either side of the meat culture constructs 106 for providing access to the fibres and improving harvesting. In particular, the spaces allow detached cells to escape the meat culture constructs 106 and spaces therebetween, improving collection of harvested cells.

In examples in which the cell culturing and harvesting are performed within the same system (without transfer of meat culture constructs 106 between different stations), the harvesting system 900 may include one or more actuators that move the meat culture constructs 106 to increase spaces between them. If the harvesting system 900 receives meat culture constructs 106 from a preceding station, then the housing 902 may include a support (e.g., a slotted top plate) that receives the meat culture constructs 106 in a spaced arrangement as illustrated.

As described below, various harvesting techniques and combinations of harvesting techniques may be applied to harvest cells or components of cells (e.g., protein) from the meat culture constructs 106. In each case, the ratio of the first dimension to the second dimension, as described above, improves the harvesting technique(s).

As mentioned above, harvesting includes two stages: (1) detachment of cells from the meat culture constructs 106 (particularly the hollow fibres), and (2) removal of the cells to different location, for example to be cleaned, processed, and collected.

Once cells are detached from the meat culture constructs 106, the cells may be collected by a cell collection system 906, which extracts fluid from the housing 902 and separates the cells.

Cells are attached to the fibres via so-called cell adhesion molecules (CAMs). These are cell surface proteins that control the interaction between cells, or between cells and the extracellular matrix (ECM). CAMs are typically divided into four families of adhesion molecules: immunoglobulin-like adhesion molecules, integrins, cadherins and selectins, some of which are calcium-dependent e.g. Cadherins, which are the major CAMs responsible for cell-cell adhesion, and other calcium independent CAMs.

The nature of cell culture to a fibre is characterised by the need for initial seeding onto a surface (cell-surface adhesion), then further proliferation and/or differentiation of cells both on the surface and into multiple tissue-like layers.

As such, the process of harvesting requires both the local detachment of cells from the surface on which they are adhered and/or from one another, then the removal away from the fibres. As used herein the term "cell detachment" and similar refers to both cell-fibre detachment and cell-cell detachment, which may be termed dissociation. It may be beneficial to harvest the cells partially by retaining the cells adhered to the surface and removing the cells adhered to other cells.

For example, in conventional mammalian cell culture, Trypsin, a proteolytic enzyme, cleaves the C-terminal side of lysine and arginine residues and breaks down the attachment proteins enabling release of cells. When using Trypsin (or similar) it is possible to assist cell detachment by applying some initial forces, for example, a sharp tap of the housing, in order to dislodge cells after the modification to the adherent protein(s).

As detailed below, various harvesting techniques may be applied to detach cells, and to remove cells (for collection). These harvesting techniques may employ one or more of physical detachment methods, chemical detachment methods, and biochemical (enzymatic) detachment methods.

In examples, harvesting is performed once the cells have reached a certain number and/or mass and/or maturity. In examples, the harvesting process may be initiated after a predetermined time period has elapsed, and/or once a predetermined maturation period has elapsed, and/or once a size and/or mass of the cells reaches a predetermined threshold. The time period may be timed from a start of the culturing method, or from the start of a maturation process (e.g., when a maturation medium is first provided).

In examples, the harvesting process may be initiated based on a detected consumption rate of nutrients within the cell culture medium (growth medium). Nutrient consumption may slow when cells reach maturity, and this change can be detected to determine when to initiate harvesting.

In examples, harvesting may be initiated based on detection of one or more markers of cell maturity. Examples of markers of cell maturation may include MyoD, PAX3, or Myogenin.

### Chemical Detachment Techniques

Chemical detachment agents may be used to reduce cell-cell adhesion and/or cell-fibre adhesion and improve detachment of cells.

In some examples, the fibres of the meat culture constructs 106 may have a surface coating that reduces adhesion of the cells. This may improve harvesting as the cells can be more easily detached. In examples, this may comprise applying fibre coatings that degrade over the cell culture period and hence release themselves along with associated cells. In examples, the fibres may comprise Chitosan coating in combination with elevated (Alkaline) pH, which may degrade during cell culturing and improve cell detachment.

In examples, the chemical detachment agent may alter the osmolality of a cell contacting liquid to induce hyper- or hypo-osmotic cell culture conditions to influence cell size and properties, which may induce detachment. For example, the chemical detachment agent may comprise a saline solution. The chemical detachment agent may be provided in the lumina of the hollow fibres or in the housing, and the fluid in the other of the hollow fibres and the housing has a higher or lower saline concentration such that osmosis occurs through the fibre walls and adhered cells.

In examples, the chemical detachment agent may remove extracellular divalent ions from calcium-dependent cell adhesion molecules. For example, the chemical detachment agent may comprise chelators such as EDTA, EGTA, citric saline, citric acid salts e.g. sodium citrate, as well as proprietary singular or blended products, for example, Chemical Dissociation Buffer.

### Biochemical Detachment Techniques

Biochemical detachment agents, in particular enzymes, may be used to reduce cell adhesion and improve cell detachment.

In examples, an enzymatic detachment agent may comprise enzymes that cleave residues on attachment proteins. In examples, the enzymatic detachment agent may comprise proteolytic enzymes otherwise known as proteases. Proteases are classed into four main mechanistic classes: aspartyl-, cysteine-, metallo- and serine-proteases.

Singular proteases or combinations of proteases may comprise animal-, microbial-, plant-, naturally- or synthetically-derived enzymes. Examples of which may include trypsin, chymotrypsin, pepsin, rennin, collagenase, microbial collagenase, actinidain, bromelain, ficain, nattokinase, papain, pronase, zingibain, as well as proprietary singular or blended products, for example, TrypLE^{™}, Detachin^{™} or Accutase^{™}.

In examples, the protease enzymatic detachment agent may be provided once at least some of the cells to be harvested have reached a mature state. In particular, mature cells with fused myotubes may be more easily detached from the fibres by the protease enzymatic detachment agent. It may therefore be advantageous for harvesting to allow the cells to reach a level of maturity in order to improve harvesting.

Similarly, the protease enzymatic detachment agent may encourage the cell to detach naturally by maturing it through its life cycle. For example, it is a known phenomena for mammalian skeletal muscle cells to spontaneously detach from substrates as they mature through to terminal differentiation. A maturation medium may be provided in the meat culturing system to mature the cells.

In more detail, as myoblasts fuse, form myotubes, and progress through maturation, changes in extracellular matrix (ECM) composition (protease-mediated breakdown of myoblast-surface hyaluronic acid, contributing to fusion; laminin secretion with the formation of syncytial myotubes) cell surface receptor expression (upregulation of laminin-binding integrins α6 β1 and α7 β1; β1D subunit replacing the β1A isoform; other ECM component-binding subunits α1 and α5 are downregulated) and internal cytoskeletal structure and composition (upregulation of actin and myosin), contributing to changes in the elastic properties, all of which culminate in changes to the attachment characteristics of differentiating myotubes. As cells mature, a contractile prestress builds within the cells (sometimes termed the 'maturation force'). This force continues to build until the contractile-adhesion balance shifts such that cells overcome receptor-ECM ligand bonds at focal adhesions, and peel from the surface of the substrate.

Therefore, in examples, the enzymatic detachment agent may be provided once a majority of the cells to be harvested have reached a mature state in order to improve harvesting.

In examples, one or more parameters are monitored to determine when the majority of the cells have reached maturity.

For example, a predetermined time period may be allowed to elapse before harvesting. The predetermined time period may be a culturing time, from the start of the seeding or culturing process. Alternatively, the predetermined time period may be a maturation time, for example a time for which a maturation medium is provided to support maturation of the cells.

In other examples, the size and/or mass of cells may be monitored to determine when to initiate harvesting. The increase of the size and mass of the cells may slow once maturation or another limit is reached (e.g., a limit of nutrient supply across the cells), indicating that the meat is ready to be harvested.

In other examples, one or more cell culture medium concentrations may be detected to monitor cell consumption. In particular, nutrient consumption (e.g., oxygen or sugars) may slow as cells approach and reach maturation, and this change in nutrient consumption may be monitored to determine when to being harvesting.

In other examples, one or more markers of cell maturation may be detected (e.g., MyoD, PAX3, or Myogenin). Once such markers are detected, or reach a threshold, harvesting may be initiated.

Enzyme activity is moderated by a number factors, such as the enzyme type, temperature, enzyme concentration, pH and fluid salt concentration. As such, it is possible to degrade proteins too rapidly and extensively such that the cell protein content is degraded, which impacts yield and hence process efficiency. Therefore, the application of specific enzymes is controlled based on factors such as the cell type, the surface type and the respective benefits of other process variables used in combination with enzymes.

### Application of Chemical and/or Biochemical Detachment Agents

Detachment agents may be applied by flowing a solution containing the agent directly into the extra-capillary space (the space within the housing surrounding the cell culture construct). Alternatively or additionally, the detachment agent can be flowed through the lumina of the hollow fibres. The detachment agent may directly flow onto the cells, or it may permeate through pores in the walls of the fibres.

In examples where the cells are cultured on the outer surfaces of the hollow fibres it is preferable to flow the detachment agent through the lumina of the hollow fibres. This provides several advantages, namely:
1. A lower quantity of enzymatic detachment agent may be used given the relatively smaller volume of the combined lumina compared to the extra-capillary volume.
2. It enables recovery and collection of any unused agent without the need to separate it from cells.
3. It would reduce modification of other agent or agent solution properties, such as temperature or pH, without it substantially impacting the cell culture environment.
4. The agent may be applied directly to the cell-surface interface, thereby enabling cell-fibre detachment without causing cell-cell dissociation.
5. It provides the ability to use a different detachment agents or removal processes in the extra-capillary space, for example a fluid flow to carry cells to a collection port outside of the meat culture construct.

### Physical Detachment and Collection Techniques

Various physical detachment techniques may be used, alone or in combination with the chemical and/or biochemical detachment techniques described above. The physical techniques may themselves contribute to (or achieve) cell detachment, or may work synergistically with the chemical/biochemical detachment agents to promote detachment. Additionally or alternatively, the physical techniques may provide collection of the detached cells, for example entrainment of the cells in a fluid flow that is directed to a collection point.

In one example the harvesting system 900 includes a detachment fluid supply system 904 for circulating fluid through the housing 902, about the meat culture constructs 106, and/or through the hollow fibres of the meat culture constructs 106. The detachment fluid supply system 904 may include one or more pumps operable to generate positive or negative pressure (suction) to generate a fluid flow. The detachment fluid supply system 904 may circulate a cell detachment fluid, which may be chemical or biochemical as described above. The cell detachment fluid may cause cells to detach from the fibres and be entrained in the fluid flow for collection.

The ratio of the first dimension to the second dimension of each meat culture construct 106 improves penetration of the cell detachment fluid into the fibre bundles, allowing more of the cells to be treated and detached, improving harvesting. In particular, harvesting may be more efficient by recovering more cells and/or taking less time to recover the cells and improving cell viability.

As shown in FIG. 9, the harvesting system 900 may include an electromagnetic emitter 908. The electromagnetic emitter 908 may irradiate the meat culture constructs 106 with electromagnetic radiation (EM radiation), for example visible light, UV light, or other EM radiation. The EM radiation may cause cells to detach from the meat culture constructs 106.

The ratio of the first dimension to the second dimension of each meat culture construct 106 improves penetration of the EM radiation into the fibre bundles, allowing more of the cells to be treated and detached, improving harvesting. In particular, harvesting may be more efficient by recovering more cells and/or taking less time to recover the cells and improving cell viability.

In other examples, the harvesting system 900 may comprise a heating and/or cooling unit for heating and/or cooling the fluid within the housing 902. The heating and/or cooling system may be operated to generate a thermal shock on the cells, which may reduce cell-fibre adhesion and/or cause them to detach from the fibres. In examples, heating the cells may degrade attachment proteins, facilitating detachment (both cell-cell detachment and cell-fibre detachment). In examples, cooling the cells may encourage contraction and hence detachment of cells (both cell-cell detachment and cell-fibre detachment).

Similarly, the harvesting system 900 may comprise an electric field generator configured to generate an electric field within the fluid in the housing 902, or along the fibres of the meat culture constructs 106. The electric field may reduce cell-fibre adhesion and/or cause them to detach from the fibres.

As shown in FIG. 9, the harvesting system 900 may include a fluid flow system 910a, 910b. The fluid flow system 910a, 910b may attach to two opposite sides of the housing 902, as shown. The fluid flow system 910a, 910b may include one or more pumps operable to generate positive or negative pressure (suction) to generate a fluid flow. The fluid flow system 910a, 910b is operable to create a fluid flow through the housing 902 in a direction parallel to the second dimensions (the smaller dimensions) of the meat culture constructs 106. This fluid flow is in a crossflow, or transmembrane direction. The fluid flow will thereby pass through the fibre bundles of the meat culture constructs 106 in the direction of the second dimensions. The fluid flow may directly shear the cells, causing them to detach and be entrained in the fluid flow. Additionally or alternatively, the fluid flow may deform or flex the fibres, causing cells to detach. The effectiveness of the fluid flow is improved by passing the fluid flow through the fibres in the direction of the fluid fibres, because the fluid can penetrate inner fibres of the meat culture constructs 106 and detached cells can more easily be entrained (not caught in other fibres within the meat culture constructs 106).

The fluid flow system 910a, 910b may include the cell collection system 906 for recovering detached cells. The fluid flow system 910a, 910b may reverse the direction of flow periodically, for example regularly. The fluid flow system 910a, 910b may direct flow into / through the cell collection system 906 so that detached and entrained cells are provided to the cell collection system 906.

The fluid provided by the fluid flow system 910a, 910b may be a harvesting solution. The harvesting solution may comprise water, phosphate buffered saline (PBS), enzyme solution in a suitable buffer, and/or chemical agents in solution. In examples, the fluid provided by the fluid flow system 910a, 910b may include a chemical or biochemical detachment agent, as described above. In examples, the fluid provided by the fluid flow system 910a, 910b may be heated or cooled to a different temperature to the meat culture constructs 106 to generate thermal shock on the cells to increase detachment.

In some examples, the meat culture constructs 106 include a central channel as described with reference to FIG. 5A. FIG. 5B, FIG. 6A, FIG. 6B, FIG. 6C, FIG. 8C, and FIG. 8D. The central channel may include a distribution tube. In such examples, the fluid flow system 910a, 910b may be fluidly coupled to the central channels / distribution tubes for driving a fluid into, or drawing a fluid out of, the central channels. This fluid flow would pass through the fibre bundles in the direction of the second dimension. The fluid flow direction may be periodically or regularly reversed.

In examples, the fluid may be a harvesting solution. The harvesting solution may comprise water, phosphate buffered saline (PBS), enzyme solution in a suitable buffer, and/or chemical agents in solution.

The ratio of the first dimension to the second dimension of each meat culture construct 106 improves penetration of the fluid flow into the fibre bundles, allowing more of the cells to be detached and entrained, improving harvesting. In particular, harvesting may be more efficient by recovering more cells and/or taking less time to recover the cells. In addition, these harvesting techniques may improve cell viability.

In examples, the fluid flow system 910a, 910b may be adapted to provide fluid flow through the lumina of the hollow fibres of the meat culture constructs 106. In such examples the fluid flow system 910a, 910b may apply continuous or intermittent hydraulic forces within the lumina to dislodge cells from the fibres (internal or external surfaces). Similarly, the fluid flow system 910a, 910b may apply pressure within the lumina of the fibres of the meat culture constructs 106 to expand the fibres and press cells out of the meat culture construct 106 through reduction of the available extra-capillary space (the spaces between the fibres).

In examples, the fluid flow applied to the meat culture constructs 106 by the fluid flow system 910a, 910b (either crossflow or through the lumina) may be periodically reversed and/or periodically stopped and started. This may increase cell detachment.

In examples, the meat culture constructs 106 may be subject to shaking, vibration, or oscillatory motion. The meat culture constructs 106 may be subject to such motions within the housing 902, or they may be transferred to a different system for application of the motion. The ratio of the first dimension to the second dimension of each meat culture construct 106 improves the detachment caused by such motion as it allows more of the cells to be detached and entrained, improving harvesting. In particular, harvesting may be more efficient by recovering more cells and/or taking less time to recover the cells. In addition, these harvesting techniques may improve cell viability.

In examples, the meat culture constructs 106 may be subject to sonication, for example ultrasonication. Sonication may be applied continuously or intermittently to agitate cells by promoting shockwaves that detach cells. The shockwaves may create cavitation or other phenomena that further improves detachment. A sonication probe may be provided in, or temporarily placed in, the housing 902 to apply sonication. In examples where the meat culture constructs 106 comprise a central channel and/or a distribution tube (as shown in FIG. 5A for example), a sonication probe may be provided in the central channel or distribution tube. The ratio of the first dimension to the second dimension of each meat culture construct 106 improves the detachment caused by such sonication because shockwaves / cavitation will be able to penetrate the fibre bundle and detach cells from inner fibres. This allows more of the cells to be detached, improving harvesting. In particular, harvesting may be more efficient by recovering more cells and/or taking less time to recover the cells. In addition, these harvesting techniques may improve cell viability.

In some examples, detached cells are allowed to settle at the bottom of the housing 902, where they can be collected.

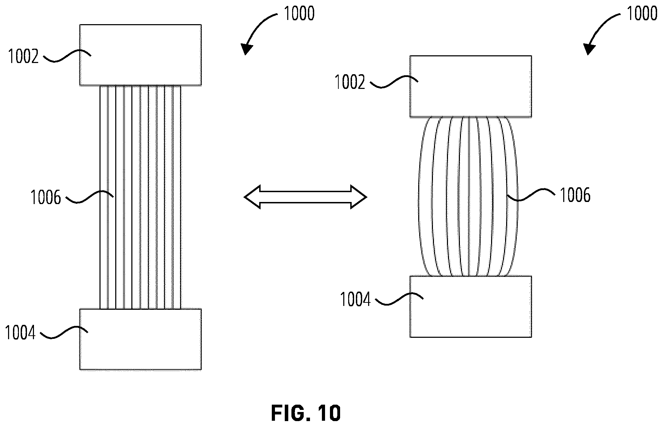

FIG. 10 illustrates a further harvesting technique that may be used within the harvesting system 900of FIG. 9. FIG. 10 illustrates a meat culture construct 1000, which may be any of the other meat culture constructs 106, 400, 500, 600, 702, 800 described above. The meat culture construct 1000 includes a first end support 1002 and a second end support 1004 and a fibre bundle 1006 extending therebetween, as with other examples.

As shown, the harvesting technique illustrated in FIG. 10 includes moving the first end support 1002 and/or the second end support 1004 relative to each other.

In the illustrated example the first end support 1002 and the second end support 1004 are moved to change a distance therebetween. In particular, the first end support 1002 is moved towards the second end support 1004 (and/or vice versa), which spreads out the fibre bundle 1006 as illustrated. The first end support 1002 and/or second end support 1004 may be moved reciprocally, for example in an oscillating motion.

The movement described above flexes the fibres of the fibre bundle 1006, which improves harvesting. In particular, the flexing increases the spaces between the fibres of the fibre bundle 1006, which improves penetration of other harvesting techniques (e.g., fluid flow, cell dissociation agents, EM radiation). In addition, flexing the fibres of the fibre bundle 1006 will itself cause cells to detach from the fibres.

In other examples, the first end support 1002 and the second end support 1004 may be moved to apply an extension force on the fibre bundle 1006. The extension force may induce a strain in the fibres, reducing their diameter and encouraging cell detachment.

In other examples, the first end support 1002 and the second end support 1004 may be moved to apply a torsional force on the fibre bundle 1006. The torsional force may deform and/or deflect the fibres, and causes contact between adjacent fibres, which may encourage cell detachment.

The first end support 1002 and/or the second end support 1004 may be mounted on moving frames or supports within the harvesting system 900 that act to move the first end support 1002 and/or the second end support 1004. The moving frame may support the meat culture constructs 1000 (i.e., hold them in the housing 902 of the harvesting system 900). The moving frame may be mounted on sliding mounts, and its movement may be powered by an actuator (e.g., a motor).

The movement of the first end support 1002 relative to the second end support 1004 may be low frequency, for example with 1 or less reciprocal motions per second. Such motion may have a magnitude sufficient to flex and spread out the fibres of the fibre bundle 1006.

In other examples, the movement of the first end support 1002 relative to the second end support 1004 may be high frequency, for example 1 or more reciprocal motions per second, for example 5 or more reciprocal motions per second, for example 10 or more reciprocal motions per second. Such a high frequency motion may have a small magnitude and create waves in the fibres that cause cell detachment.

The ratio of the first dimension to the second dimension of the meat culture construct 1000 improves the flexing of the fibre bundle 1006 because there will be fewer collisions between fibres. In addition, the ratio improves recovery of detached cells because fewer cells would be caught in the fibre bundle, so detached cells can escape the meat culture constructs 106 for collection.

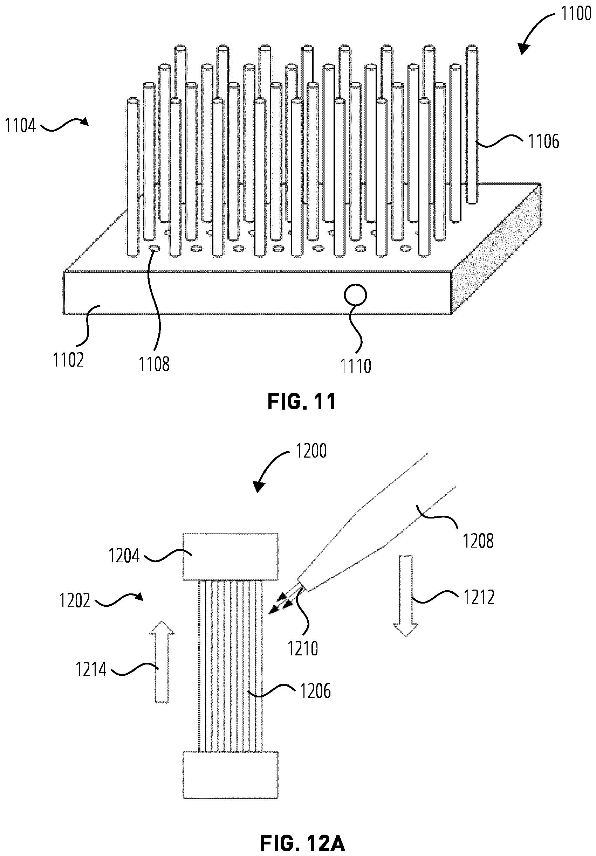

FIG. 11 illustrates a further harvesting technique that may be used within the harvesting system 900of FIG. 9. FIG. 11 illustrates a further example meat culture construct 1100, which may be any of the other meat culture constructs 106, 400, 500, 600, 702, 800, 1000 described above. FIG. 11 shows only one end of the meat culture construct 1100. The meat culture construct 1100 includes an end support 1102, and a second end support (not illustrated) may be provided at the non-illustrated end of the meat culture construct 1100. The meat culture construct 1100 includes a fibre bundle 1104 having a plurality of fibres 1106 extending from the end support 1102 (to the other end support), as with other examples.

In this example, the end support 1102 includes a plurality of fluid outlets 1108. The fluid outlets 1108 are interspersed with the fibres 1106. The end support 1102 may include a manifold arrangement for connecting the fluid outlets 1108 to a different fluid port to the fibres 1106. For example, the fluid outlets 1108 may be connected to a side port 1110 in the end support 1102, as shown. The detachment fluid supply system 904 and/or the fluid flow system 910a, 910b of the harvesting system 900 may be connected to the fluid outlets 1108 for providing a fluid thereto.

During harvesting, a fluid can be pumped through the fluid outlets 1108 to create a shear flow over the fibres 1106 that would detach cells from the fibres 1106. The fluid flow through fluid outlets 1108 may detach cells and/or flex the fibres 1106 to cause detachment, and may entrain detached cells.

In examples, the fluid may be a harvesting solution. The harvesting solution may comprise water, phosphate buffered saline (PBS), enzyme solution in a suitable buffer, and/or chemical agents in solution. In examples, gaseous bubbles may be created at fluid outlets 1108. For example, air, nitrogen, carbon dioxide, and/or oxygen may be pumped to the fluid outlets 1108. Such bubbles will promote cell detachment by shearing and/or scouring cells from the fibres.

The ratio of the first dimension to the second dimension of each meat culture construct 106 improves recovery of the detached cells because fluid flowing from fluid outlets 1108 would have improved penetration of the fibre bundle 1104 and fewer detached cells would be caught in the fibre bundle 1104.

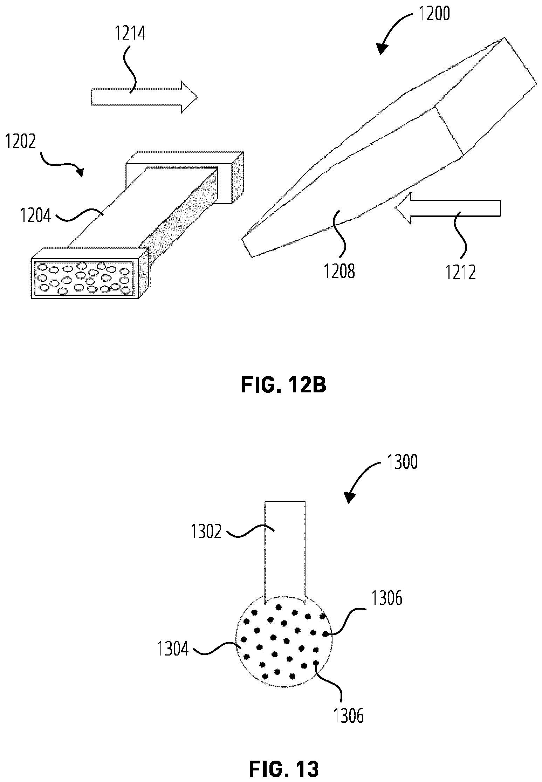

FIG. 12A and FIG. 12B illustrate a further harvesting technique that may be used within the harvesting system 900 of FIG. 9. FIG. 12A and FIG. 12B illustrate a fluid knife harvesting system 1200 being used to harvest cells from a meat culture construct 1202, which may be any of the other meat culture constructs 106, 400, 500, 600, 702, 800, 1000, 1100 described above.

The meat culture construct 1202 includes end supports 1204 and fibres 1206 extending between the end supports 1204.

The fluid knife harvesting system 1200 comprises a fluid knife 1208 with an elongate nozzle 1210 from which a laminar fluid flow is generated. The fluid flow is directed towards the fibres 1206 of the meat culture construct 1202 and acts to detach cells from the fibres 1206. The fluid flow may directly detach cells (e.g., shear or scour the cells from the fibres), and/or the fluid flow may flex the fibres 1206, causing cells to detach. Detached cells can be entrained in the fluid flow for collection.

The ratio of the first dimension to the second dimension of the meat culture construct 1202 improves penetration of the laminar fluid flow into the fibres 1206, allowing more of the cells to be detached and entrained, improving harvesting. In particular, harvesting may be more efficient by recovering more cells and/or taking less time to recover the cells. Cell viability may also be improved.

In examples, the fluid may be a harvesting solution. The harvesting solution may comprise water, phosphate buffered saline (PBS), enzyme solution in a suitable buffer, and/or chemical agents in solution. In examples, the fluid may be a gas, such as nitrogen, carbon dioxide, and/or oxygen.

In the examples of FIG. 12A and FIG. 12B the fluid knife 1208 is moved in direction of arrow 1212, and/or the meat culture construct 1202 is moved in the direction of arrow 1214 (and/or vice versa) to generate relative movement of the fluid knife 1208 and the meat culture construct 1202.

In the example of FIG. 12A the fluid knife 1208 is oriented with the elongate nozzle 1210 perpendicular to the direction of the fibres 1206 and moved in a direction parallel to the fibres 1206. In the example of FIG. 12B the fluid knife 1208 is oriented with the elongate nozzle 1210 parallel to the fibres 1206 and moved in a direction perpendicular to the fibres 1206.

FIG. 13 illustrates a further harvesting technique that may be used within the harvesting system 900 of FIG. 9. FIG. 13 illustrates a rotating ball device 1300 that can be used with a meat culture construct having a central channel and optionally also a distribution tube, such as any of those described with reference to FIG. 5A, FIG. 5B, FIG. 6A, FIG. 6B, FIG. 6C, FIG. 8C, and FIG. 8D.

The rotating ball device 1300 has a shaft 1302 and a ball 1304 mounted to the end of the shaft 1302. In one example the ball 1304 is rotatably mounted to the shaft 1302 to rotate about the axis of the shaft 1302. In another example, the shaft 1302 is rotatably mounted to a further shaft and the shaft 1302 and ball 1304 rotate together.

The ball 1304 includes a plurality of fluid outlets 1306. When fluid pressure is applied to the inside of the ball 1304, through the shaft 1302, fluid jets are generated at the fluid outlets 1306. The fluid pressure may also cause the ball 1304 (and optionally the shaft 1302) to rotate so that the directions of the fluid jets vary.

During use, the rotating ball device 1300 may be passed into the central channel or distribution tube of the meat culture construct and moved along, for example back and forth within, the central channel or distribution tube. The fluid jets will impact the fibres in the direction of the second dimension of the fibre bundle and act to detach cells therefrom. The fluid jets may directly detach cells (e.g., by shearing or scouring cells from the fibres), or the fluid jets may flex the fibres, causing cells to detach.

In examples, the fluid may be a harvesting solution. The harvesting solution may comprise water, phosphate buffered saline (PBS), enzyme solution in a suitable buffer, and/or chemical agents in solution. In examples, the fluid may be a gas, such as air, nitrogen, carbon dioxide, and/or oxygen.

In other examples, neither the end support ball 1304 or the shaft 1302 rotate and the ball 1304 acts as a water jet with individual jets having a fixed orientation with respect with the ball 1304, but moving with respect to the meat culture construct due to movement of the rotating ball device 1300 within the meat culture construct.

The ratio of the first dimension to the second dimension of the meat culture constructs improves penetration of the fluid jets into the fibres, allowing more of the cells to be detached and entrained, improving harvesting. In particular, harvesting may be more efficient by recovering more cells and/or taking less time to recover the cells.

In examples, a scraper may be used to physically scrape cells from the fibres. If the fibres are arranged in a square or rectangular shape then the scraper may be a flat scraper that is moved along one or more sides of the cell culture construct to scrape cells from the fibres. Alternatively, the scraper may be tubular with an inward facing scraping edge to scrape from more than one side simultaneously. In examples where the cell culture construct comprises fibres arranged annularly, the scraper may be arranged to scrape internally and/or externally.

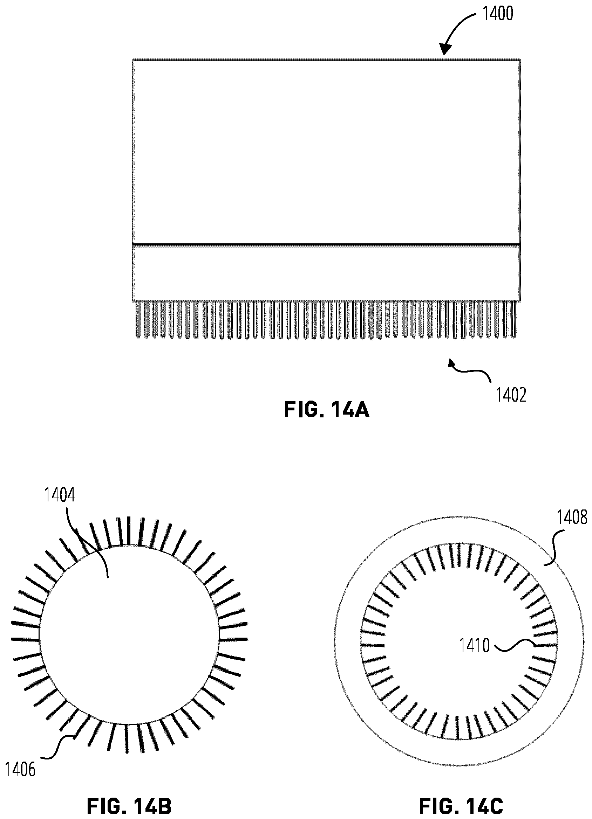

FIG. 14A illustrates a further harvesting technique that may be used within the harvesting system 900 of FIG. 9. FIG. 14A illustrates a harvesting comb 1400 that can be used with any of the meat culture constructs 106, 400, 500, 600, 702, 800, 1000, 1100, 1202 described above.

The harvesting comb 1400 includes a plurality teeth 1402 extending from one side. The teeth 1402 are parallel and regularly spaced. The teeth 1402 have a length that is at least half of the second dimension of the meat culture construct (and so the teeth 1402 penetrate to the centre of the fibre bundle). The harvesting comb 1400 can be used to physically scrape cells from the fibres in order to detach them for harvesting. The teeth 1402 of the harvesting comb 1400 can be pushed into the fibre bundle, and the harvesting comb 1400 can be moved along the meat culture construct in a direction parallel to the fibres to scrape cells. The harvesting comb 1400 may be used from one side, or from opposite sides, preferably penetrating the fibre bundle in the direction of the second dimension. The harvesting comb 1400 may be moved reciprocally along the meat culture construct.

The ratio of the first dimension to the second dimension of the meat culture constructs improves penetration of the teeth 1402 into the fibres, allowing more of the cells to be scraped and detached, improving harvesting. In particular, harvesting may be more efficient by recovering more cells and/or taking less time to recover the cells.

FIG. 14B and FIG. 14C illustrate similar concepts for circular / annular meat culture constructs. The internal comb 1404 of FIG. 14B can be passed through the central channel of the meat culture construct and the outwardly-facing teeth 1406 will act to scrape cells from the fibres from within the central channel. Similarly, the external comb 1408 of FIG. 14C can be passed over a circular meat culture construct and the inwardly-facing teeth 1410 will act to scrape cells from the fibres. Similar combs can be provided for other shapes of meat culture constructs.

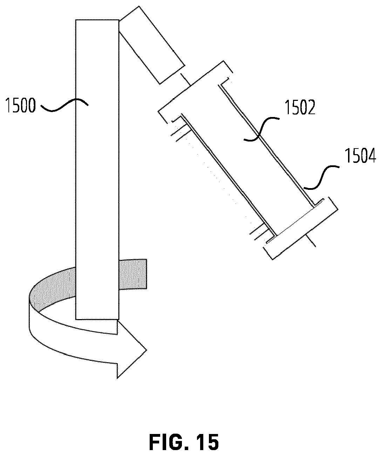

FIG. 15 illustrates a centrifuge 1500 that may be used to harvest and/or cells from a meat culture construct 1502. The meat culture construct 1502 may be provided in a housing 1504 (which in examples may be the housing of the meat culturing system as described above) and attached to a centrifuge 1500. The centrifuge rotates and centrifugal forces promote cell detachment and movement to the end of the housing 1504, where they can be collected. Centrifugation using the centrifuge 1500 may be performed after other cell detachment techniques described above, and may in particular improve collection of detached cells.

In examples, the centrifuge 1500 is configured to rotate the meat culture construct 1502 about a central axis (an axis central to the meat culture construct 1502), urging cells outwardly and out of the fibres.

In other examples, the centrifuge 1500 is configured to rotate the meat culture construct 1502 about an offset axis to urge cells towards a particular location of the housing 1504, for example a collection port.

In other examples, the centrifuge 1500 is configured to rotate the meat culture construct 1502 about an axis intersecting a longitudinal axis of the meat culture construct 1502, as shown in FIG. 15. In this example, detached cells would be urged towards the end of the housing 1504 (specifically a lower end) where they can be collected.

In each example, the ratio of the first dimension to the second dimension of the meat culture constructs allows more of the cells to escape from the fibre bundle in the meat culture construct 1502, improving harvesting.

As described above, various harvesting techniques can be used to detach and remove cells from the meat culture constructs. These techniques may be used in various combinations to improve detachment and collection.

In one example, a cell detachment agent (e.g., chemical or biochemical (enzymatic)) may be used to detach cells, and a fluid flow (e.g., as generated by the fluid flow system 910a, 910b described above) may be used to remove the detached cells to the cell collection system 906.

In another example, a cell detachment agent (e.g., chemical or biochemical (enzymatic)) may be used to detach cells and/or reduce cell adhesion, and one or more of the physical harvesting techniques described above may be used to detach more cells. A fluid flow (e.g., as generated by the fluid flow system 910a, 910b described above) may be used to remove the detached cells to the cell collection system 906.

In another example, cells are cultured on an external surface of the fibres of the meat culture construct. In this example, an enzymatic detachment agent is flowed through the lumen of the fibres under pressure. This can provide two effects: (1) the enzymatic detachment agent permeates through the fibres and reduces cell-fibre adhesion; and (2) the pressure of the fluid flow pushes cells away from the fibres and generates an entrainment flow carrying the cells away from the meat culture construct. The fluid flow may be directed across the meat culture constructs, in the direction of the second dimension. The entrainment flow may be directed to a cell collection system.

In another example, a protease enzymatic detachment agent is first provided to reduce cell-fibre adhesion. Following this, a fluid flow with reciprocating direction (reversal) and/or pressure spikes is provided through the housing and/or through the hollow fibres of the meat culture construct to dislodge the cells and complete detachment. Optionally, the meat culture construct may be vibrated or subject to other motion to further promote cell detachment. After cells have been detached a unidirectional fluid flow may be provided to convey detached cells to a cell collection system.

In another example, a cell detachment agent is first provided to reduce cell-fibre adhesion. Following this, a fluid flow with reciprocating direction (reversal) and/or pressure spikes is provided through the housing and/or through the hollow fibres of the meat culture construct to dislodge the cells and complete detachment. Optionally, the meat culture construct may be vibrated or subject to other motion to further promote cell detachment. After cells have been detached a unidirectional fluid flow may be provided to convey detached cells to a cell collection system.

In another example, an enzymatic detachment agent is provided to reduce cell -fibre adhesion and/or to detach cells. Once a period of time has elapsed, sonication (e.g., ultrasonication) can be applied to generate shockwaves to further dislodge and detach cells. The sonication may be applied with a frequency between 20 kHz and 180 kHz. A fluid flow may then be provided to remove cells to a cell collection system.

In another example, the meat culture constructs may be heated. Heating may degrade attachment proteins, promoting detachment. Further heating may cause collagen within the cultured meat to stiffen (akin to cooking). Following heating, the cells may be more easily removed from the fibres by scraping or combing (e.g., using an air scour technique, or a scraper or comb as described above), akin to removing cooked meat from a kebab skewer.

In another example, a protease enzyme detachment agent may be provided to reduce cell adhesion and cause cells to detach. After a period of time the meat culture construct may be centrifuged to further detach cells and collect them at an end of the housing.

In another example, the method of FIG. 10 (moving the first end support 1002 and/or second end support 1004 to spread apart the fibre bundle 1006) may be combined with application of a chemical or biochemical detachment agent in the extra-capillary space. The increased spaces between the fibres would improve penetration of the detachment agent into the fibre bundle 1006.

## Claims

1. A meat culture construct for a meat culturing system, the meat culturing system having a housing adapted to receive the meat culture construct,
wherein the meat culture construct comprises a plurality of fibres and an end support in which ends of the plurality of fibres are embedded such that the plurality of fibres extend from the end support and into the housing when the meat culture construct is received in the housing of the meat culturing system, and
wherein at a plane through the end support the plurality of fibres are arranged in a fibre distribution defined by an envelope containing the plurality of fibres, wherein the envelope has a first dimension and a second dimension, and wherein a ratio of the first dimension to the second dimension is at least 2:1 in order to enhance fluid access to fibres within the fibre distribution.

2. The meat culture construct of claim 1, wherein the ratio of the first dimension to the second dimension is at least 2.5:1, for example at least 3:1, for example at least 3.5:1, for example at least 4:1, for example at least 5:1, for example at least 10:1, for example at least 20:1.

3. The meat culture construct of claim 1 or 2, wherein the envelope is rectangular.

4. The meat culture construct of claim 1 or 2, wherein the envelope is an annulus formed between an outer boundary and an inner boundary, the second dimension being a distance between the inner boundary and the outer boundary, and wherein the first dimension is a length of the inner boundary or the outer boundary.

5. The meat culture construct of any one of claims 1 to 4, wherein the plurality of fibres are hollow fibres, each hollow fibre comprising a lumen, and wherein the first end support comprises a fluid port in fluid communication with lumina of the plurality of hollow fibres.

6. The meat culture construct of any one of claims 1 to 5, wherein the end support comprises a plurality of fluid outlets for providing a flow of fluid along the meat culture construct, parallel to the plurality of fibres.

7. A meat culturing system comprising a housing and one or more of the meat culture constructs of any one of claim 1 to claim 6 received in the housing.

8. A harvesting system comprising a housing and one or more of the meat culture constructs of any one of claim 1 to claim 6 received in the housing.

9. The harvesting system of claim 8, further comprising a fluid supply system arranged to generate a fluid flow through the housing in a direction parallel to the second dimension of the one or more meat culture constructs.

10. A harvesting method for harvesting muscle cells from the meat culture construct of the harvesting system of claim 8 or claim 9, the harvesting method comprising detaching the muscle cells from the plurality of fibres, and collecting the detached muscle cells.

11. The harvesting method of claim 10, wherein detaching the muscle cells comprises applying a physical detachment technique.

12. The harvesting method of claim 11, wherein the physical detachment technique comprises providing a fluid flow in a direction parallel to the second dimension of the one or more meat culture constructs.

13. The harvesting method of claim 11 or claim 12, wherein the physical detachment technique comprises heating and/or cooling the one or more meat culture constructs.

14. The harvesting method of any one of claims 11 to 13, wherein the physical detachment technique comprises applying motion to at least a part of the one or more meat culture constructs, for example vibration, shaking or oscillatory motion.

15. The harvesting method of any one of claims 10 to 14, comprising providing a chemical or biochemical detachment agent to the or each meat culture construct and providing a fluid flow to detach cells from the plurality of fibres.
